# EUROPEAN PATENT SPECIFICATION

(11) **EP 4 384 082 B1**
(45) Date of publication and mention of the grant of the patent: **06.05.2026**
(21) Application number: 22757361.5
(22) Date of filing: 02.08.2022
(51) Int. Cl.: A61B 17/06, A61L 17/14, A61L 17/00, A61B 17/00, A61B 90/00

(54) **SYSTEMS, KITS, AND METHODS FOR COATING SUTURES WITH MEDICINAL COMPOUNDS IMMEDIATELY PRIOR TO SUTURE IMPLANTATION**
SYSTEME, KITS UND VERFAHREN ZUR BESCHICHTUNG VON NAHTMATERIAL MIT MEDIZINISCHEN VERBINDUNGEN UNMITTELBAR VOR DER NAHTIMPLANTATION
SYSTÈMES, KITS ET PROCÉDÉS POUR RECOUVRIR DES SUTURES AVEC DES COMPOSÉS MÉDICINAUX IMMÉDIATEMENT AVANT L'IMPLANTATION DE SUTURES

(30) Priority: 13.08.2021 US 202163232696 P
(43) Date of publication of application: 19.06.2024
(73) Proprietor: Ethicon, Inc., Raritan, NJ 08869 (US)
(72) Inventor: SCALZO, JR., Howard, Raritan, New Jersey 08869 (US); POKROPINSKI, JR., Henry, Raritan, New Jersey 08869 (US); KRIKSUNOV, Leo, Raritan, New Jersey 08869 (US)
(74) Representative: Carpmaels & Ransford LLP
(86) International application number: PCT/IB2022/057165
(87) International publication number: WO 2023/017364

(56) References cited:
- EP-A1- 2 712 555
- WO-A1-2017/075548
- WO-A2-2008/045338

## Description

### FIELD

The field of art to which this invention relates is absorbable or non-absorbable medical devices, such as monofilament sutures or multifilament such as braided sutures, more specifically surgical sutures configured for coating or impregnating with beneficial agents or medicants immediately prior to the suture installation into the tissue.

### BACKGROUND

Surgical sutures and attached surgical needles are well known in the art for use in a variety of conventional surgical procedures. For example, such sutures may be used to approximate tissue about incisions or lacerations in epidermal layers and underlying fascia layers, join blood vessel ends, attach tissue to medical devices such as heart valves, repair body organs, repair connective tissue, etc. Conventional surgical sutures may be made from known biocompatible materials, particularly synthetic and natural biocompatible polymeric materials, which may be non-absorbable or absorbable. Examples of synthetic non-absorbable polymeric materials useful to manufacture non-absorbable sutures include polyesters, polyolefins, polyvinylidene fluorides and polyamides. Further examples of non-absorbable materials are polyethylene, polypropylene, nylon, and similar polymers. Examples of synthetic absorbable polymeric materials useful to manufacture absorbable sutures include polymers and copolymers made from lactones such as the lactides, glycolide, p-dioxanone, epsilon-caprolactone, and trimethylene carbonate. The term absorbable is meant to be a generic term, which may also include implantable devices that bioabsorbable, resorbable, bioresorbable, degradable or biodegradable in the living body or tissue. The term non-absorbable is meant for implantable devices that are permanently installed in the living body or tissue.

Sutures are preferred by surgeons for use in many surgical procedures because of several advantages and properties possessed by such sutures. Absorbable sutures must be capable of providing the desired tensile strength in vivo for a sufficient period of time to allow for effective tissue healing. Wound healing is dependent on the nature of the specific tissue as well as the healing characteristics of the individual undergoing the surgical procedure. For example, poorly vascularized tissue is likely to heal more slowly than highly vascularized tissue; likewise, diabetic patients and the elderly tend to heal more slowly as well. There are thus opportunities to provide suture materials that can match the healing characteristics of a variety of wounds. Any implant, such as a suture, appears as a foreign body to the patient's immune system. In addition, it is known that implantable medical devices, including sutures, may provide a platform for the attachment of bacteria and the subsequent formation of bacterial biofilms. It was found beneficial for sutures to have antimicrobial properties.

Surgical sutures are designed to have the requisite physical characteristics to assure desirable and efficacious in vivo behavior. Absorbable sutures need to retain appropriate tensile strength during the required healing period; this is typically characterized as breaking strength retention (BSR). In order to obtain the required design properties, it is necessary to provide absorbable polymers and manufacturing processes that will yield absorbable sutures with the required properties.

Likewise, the retention of mechanical properties, including, e.g. tensile strength and knot strength, post-implantation, is often a very important and critical feature of an absorbable medical device. The device must retain mechanical integrity until the tissue has healed sufficiently. In some bodily tissues, healing occurs more slowly, requiring an extended retention of mechanical integrity. As mentioned earlier, this is often associated with tissue that has poor vascularization. Likewise, there are other situations in which a given patient may be prone to poor healing, e.g., the diabetic patient.

U.S. Patent No. 8997978 "Medical device package" discloses a medical device package comprising: a medical device; at least one solid self-contained agent in a form selected from the group consisting of capsule, tablet, pellet, and pressed powders; a container having an area configured for receiving the medical device, and the at least one solid self-contained agent; and a port for permitting the sterile passage of a contact material between the outside of the container and the at least one solid self-contained agent.

U.S. Patent Publication No. 2009/0209031A1 "MEDICAL DEVICE PACKAGE" discloses a package for a medical device, the package capable of sustaining viable cells, the package comprising: a first container configured to receive a medical device and to sustain at least one viable cell; a fluid port in communication with the first container for allowing sterile passage of an agent to the medical device and for maintaining cell viability.

U.S. Patent Publication No. 2007/0170080A1 "Medical device package" discloses a package comprising: a sealable pouch for a medical device; and a sealed port positioned adjacent a periphery of the sealable pouch, for permitting the passage of at least one agent to the medical device contained therein from outside the sealable pouch.

U.S. Patent No. 10,632,225B2 "Surgical sutures incorporated with stem cells or other bioactive materials" discloses a method of making a surgical suture or an elongate linear tissue scaffold having an exterior surface and interior core comprised of a matrix of interwoven filaments or a porous monofilament, wherein the exterior surface comprises constrictions at intervals along the longitudinal axis and wherein a concentration of a bioactive material selected from the group consisting of biological cells and therapeutic agents is dispersed throughout said interior core, said method comprising the steps of: compressing or otherwise manipulating said matrix along or around a longitudinal axis so as to deform the interstices between interwoven filaments or the pores of the monofilament to a first size and shape sufficient to permit the migration of said bioactive material from a surrounding media, across said exterior surface, and into said interior core; and manipulating said bioactive material-containing matrix along or around said longitudinal axis so as to shrink said interstices or pores to a second size and shape sufficient to restrict the migration of said bioactive material from said interior core across said exterior surface.

U.S. Patent Publication No. 2006/0287676A1 "Method of intra-operative coating therapeutic agents onto sutures, composite sutures and methods of use" discloses a device for coating a suture, comprising: a) a vessel having an open end, a closed end and an inner wall, b) a porous body enclosing the open end of the vessel; c) a breakable seal disposed upon the inner wall of the vessel defining a first chamber and a second chamber, d) a fluid contained within the second chamber, and e) a therapeutic agent contained within or on the porous body.
EP2712555A1 relates to packaging and dispenser embodiments for sutures including bi-directional sutures which are used to reduce catching and tangling of the sutures.
WO2017075548 relates to systems, devices and methods which are said to improve safety and efficiency in an operating room comprising providing a suture package that holds suture needles and needle receptacles for storing used needles.
WO2008045338 relates to a medical device package including a container for receiving a medical device having an area configured for storing at least one agent and a port for permitting the passage of a contact material between the outside of the container and the area configured for storing the agent.

It is known to include certain medicants or beneficial agents, such as antimicrobials, in or on sutures or other medical implants such as hernia meshes or the like, for rapid release or for release over extended periods of time in vivo, so as to reduce the likelihood of post-surgical infections.

The current solutions to apply various medically useful agents or medicants to implantable sutures are related, for example, to (i) medicant-containing coatings that are applied via coating of the suture in a liquid phase (medicant solutions in solvents, incl. aqueous, ethyl acetate, etc.), with consequent drying, packaging, and sterilization of the coated suture; and (ii) transferring the medicants to coat the suture via vapor phase transfer (e.g. sublimating triclosan) from inside the suture package container. The current methods of coating the sutures have some limitations, including: These methods might be limited to one agent only or to medicants that can be applied as a coating and then stored long term with the suture as a coating prior to suture use. Further, these techniques are challenging to be used with some medicants, such as biologics, such as proteins, such as antibodies. Also, there are limited types of the sterilization techniques that can be applied to such coated sutures.

Further, additions of large amounts of a medicant needed to provide substantial antibacterial properties to a suture, either by in advance incorporation into the suture material itself, or via an advance surface coating of the suture, can be difficult to apply, process, and package. **In** particular, it can be challenging to utilize an appropriate sterilization method that can be applied to the suture containing the medicant, due to different interactions of the medicant and the suture material itself with the sterilizing modalities, including sterilizing process and or sterilizing chemistry. Certain medicants can be rendered ineffective when exposed to suture sterilization processes. Certain medicants can also degrade when exposed to suture sterilization processes, thus the medicant is incompatible with the suture sterilization process.

Additionally, for some medicants, there could be a mismatch of shelf life between the suture and the medicant, for example the suture can have a shelf life of 5 years, but the medicant could have a lesser shelf life, such as e.g. 1 year. Further, there could be a mismatch of storage conditions between the suture and the medicant, for example the suture is can be stored at room temperature, but the medicant needs to be refrigerated, etc.

### SUMMARY

The present invention is defined by the features of the independent claims. Embodiments of the invention are defined in the dependent claims.

Presented is an implantable medical device, such as a surgical suture, attached to a needle for insertion into the tissue and passing the suture though the tissue, the surgical suture configured for coating with beneficial agents or medicants immediately prior to the suture installation into the tissue. The suture is contacted with the beneficial medicant within seconds to minutes prior to installation into the tissue, such as from about 5 seconds to about 120 minutes prior to installation. Preferably, the suture is contacted with the beneficial medicant after the sterilization treatment of the suture and of the medicant, most preferably from about 5 seconds to about 30 minutes prior to installation into the tissue.

The inventive suturing system comprises an elongated flexible suture having a connecting end attached to a needle and an opposing free end, said suture co-packaged with a pierceable container containing a medicant solution, wherein said pierceable container further contains a sponge wetted with said medicant solution. The pierceable container can optionally comprise a pierceable septum. In another aspect, there is provided a method of coating the suture comprising the steps of: Gripping the needle manually or with a suture gripper; Piercing said pierceable container with said needle; Entering said pierceable container with said needle and pulling said needle through said pierceable container; exiting said pierceable container with said needle in a different spot; Pulling said suture through said pierceable container behind said needle, thus exposing said suture to said medicant solution inside said pierceable container; Removing all suture from said pierceable container thus forming suture wetted with said medicant solution and retaining at least some medicant on said suture; Optionally allowing at least a portion of said solution to evaporate.

### BRIEF DESCRIPTION OF THE DRAWINGS

The present disclosure is susceptible to various modifications and alternative forms, specific exemplary implementations thereof have been shown in the drawings and are herein described in detail. It should be understood, however, that the description herein of specific exemplary implementations is not intended to limit the disclosure to the particular forms disclosed herein.

This disclosure is to cover all modifications and equivalents as defined by the appended claims. It should also be understood that the drawings are not necessarily to scale, emphasis instead being placed upon clearly illustrating principles of exemplary embodiments of the present invention. Moreover, certain dimensions may be exaggerated to help visually convey such principles. Further where considered appropriate, reference numerals may be repeated among the drawings to indicate corresponding or analogous elements. Moreover, two or more blocks or elements depicted as distinct or separate in the drawings may be combined into a single functional block or element. Similarly, a single block or element illustrated in the drawings may be implemented as multiple steps or by multiple elements in cooperation.

The forms disclosed herein are illustrated by way of example, and not by way of limitation, in the figures of the accompanying drawings and in which like reference numerals refer to similar elements and in which:
FIGs. 1A-1C present schematic side cross-sectional views of suture and needle.
FIG. 2A shows a schematic side cross-sectional view of an embodiment of pierceable container with medicant
FIG. 2B shows a schematic side cross-sectional view of an embodiment of pierceable container with medicant.
FIG. 2C shows a schematic side cross-sectional view of an embodiment of pierceable container with medicant and connected suture in suture packaging box mounted on solid support in proximity to each other.
FIG. 2D shows a schematic side cross-sectional view of an embodiment of pierceable container with medicant co-packaged with connected suture in a suture packaging box, both enclosed in a unified package, forming a kit for applying beneficial medicant to suture.
FIG. 2E shows a schematic side cross-sectional view of an embodiment of pierceable container with medicant, the container closed with a pierceable septum.
FIG. 2F shows a schematic side cross-sectional view of an embodiment of pierceable container with medicant as a liquid solution distributed in a sponge.
FIG. 2G shows a schematic side cross-sectional view of pierceable container with medicant as a liquid solution distributed in a sponge, with the container closed with a pierceable septum.
FIG. 3A shows a schematic side cross-sectional view of an embodiment of the pierceable container in use during needle piercing the wall of the container from outside.
FIG. 3B shows a schematic side cross-sectional view of an embodiment of the pierceable container in use during needle piercing the wall of the container from outside and needle exiting container, piercing container wall from inside.
FIG. 3C shows a schematic side cross-sectional view of an embodiment of the pierceable container in use during pulling suture through the container.
FIG. 3D shows a schematic side cross-sectional view of an embodiment of the present invention, with pierceable container in use during needle piercing the septum of the container from outside and needle exiting the septum from inside the container.
FIG. 4A shows a schematic side cross-sectional view of an embodiment of frangible container with medicant.
FIG. 4B shows a schematic side cross-sectional view of an embodiment of frangible container with medicant mounted inside porous sponge.
FIG. 4C shows a schematic side cross-sectional view of an embodiment of frangible container with medicant mounted in contact with porous sponge.
FIG. 4D shows a schematic side cross-sectional view of an embodiment of frangible container with medicant mounted inside porous sponge, with solid support having an indentation or cut-out or basin.
FIG. 4E shows a schematic side cross-sectional view of an embodiment of frangible container with medicant mounted not inside porous sponge, but within the basin.
FIG. 4F shows a schematic side cross-sectional view of an embodiment of frangible container with medicant mounted inside porous sponge co-packaged in a unified package with connected suture in a suture packaging box, forming a kit for applying beneficial medicant to suture.
FIG. 5A shows a schematic side cross-sectional view of an embodiment of frangible container with medicant mounted inside porous sponge in use.
FIG. 5B shows a schematic side cross-sectional view of an embodiment of frangible container with medicant mounted inside porous sponge in use as frangible container is deformed and crushed.
FIG. 5C shows a schematic side cross-sectional view of an embodiment of frangible container with medicant mounted inside porous sponge in use as frangible container is releasing liquid medicant that is being rapidly wicked into the sponge.
FIG. 5D shows a schematic side cross-sectional view of an embodiment of frangible container with medicant in use. The container is mounted inside the basin proximal to the sponge.
FIG. 5E shows a schematic side cross-sectional view of an embodiment of frangible container with medicant in use. The frangible container is mounted inside the basin proximal to the sponge, and is releasing liquid medicant into the basin, with the medicant being rapidly wicked into the sponge.
FIG. 5F shows a schematic side cross-sectional view of an embodiment of frangible container in use, with frangible container represented by a plurality of frangible, easily breakable capsules or microcapsules with medicant mounted inside porous sponge.
FIG. 6A shows a schematic side cross-sectional view of an embodiment of the coating of suture via pulling suture through a sponge containing liquid. Needle is shown entering the sponge by piercing the sponge from outside.
FIG. 6B shows a schematic side cross-sectional view of an embodiment of the coating of suture via pulling suture through sponge containing liquid. Needle is shown exiting the sponge.
FIG. 6C shows a schematic side cross-sectional view of an embodiment of the coating of suture via pulling suture through sponge containing liquid. Suture is shown being pulled though the sponge behind the needle.
FIG. 7A shows a schematic side cross-sectional view of an embodiment showing suture positioned in a slit formed in the sponge.
FIG. 7B shows a schematic perspective view of an embodiment showing suture positioned in a slit formed in the sponge.
FIG. 7C shows a schematic perspective view of an embodiment showing suture positioned in a slit formed in the sponge.
FIG. 7D shows a schematic perspective view of an embodiment showing a non-linear slit formed in the sponge.
FIG. 8A shows a schematic side cross-sectional view of an embodiment of frangible container with a solvent. The sponge is coated throughout or impregnated with a dry beneficial agent or medicant, at least partially soluble in the solvent.
FIG. 8B shows a schematic side cross-sectional view of an embodiment of frangible container with a solvent mounted inside porous sponge on a solid support. The sponge is coated throughout or impregnated with a dry beneficial agent or medicant, at least partially soluble in solvent.
FIG. 8C shows a schematic side cross-sectional view of an embodiment of frangible container with a solvent mounted in contact with porous sponge. The sponge is coated throughout or impregnated with a dry beneficial agent or medicant, at least partially soluble in the solvent.
FIG. 8D shows a schematic side cross-sectional view of an embodiment of frangible container with a solvent mounted inside porous sponge, with solid support having an indentation or cut-out or basin, and the sponge and the frangible container at least partially positioned in the basin. The sponge is coated throughout or impregnated with a dry beneficial agent or medicant, at least partially soluble in the solvent.
FIG. 8E shows a schematic side cross-sectional view of an embodiment of frangible container with solvent mounted not inside the porous sponge, but within the basin. The sponge is mounted at least partially in the basin. The sponge is coated throughout or impregnated with a dry beneficial agent or medicant, at least partially soluble in the solvent.
FIG. 9A shows a schematic side cross-sectional view of an embodiment of frangible container with a solvent mounted inside the porous sponge in use. The sponge is coated throughout or impregnated with dry beneficial agent or medicant, at least partially soluble in solvent.
FIG. 9B shows a schematic side cross-sectional view of an embodiment of frangible container with solvent mounted inside porous sponge in use, as frangible container is deformed and crushed. The sponge is coated throughout or impregnated with dry beneficial agent or medicant, at least partially soluble in solvent.
FIG. 9C shows a schematic side cross-sectional view of an embodiment of frangible container with solvent mounted inside porous sponge in use, as frangible container is releasing solvent that is rapidly wicked into sponge. The sponge is coated throughout or impregnated with dry beneficial agent or medicant, at least partially soluble in solvent.
FIG. 9D shows a schematic side cross-sectional view of an embodiment of frangible container with solvent mounted alongside but not inside porous sponge, in use, with both frangible container and the sponge positioned at least partially inside the basin. The sponge is coated throughout or impregnated with dry beneficial agent or medicant, at least partially soluble in solvent.
FIG. 9E shows a schematic side cross-sectional view of an embodiment of frangible container with solvent in use, as frangible container is releasing solvent into basin, with the sponge rapidly wicking solvent from the basin. The sponge is coated throughout or impregnated with dry beneficial agent or medicant, at least partially soluble in solvent.
FIG. 10A shows a schematic side cross-sectional view of an embodiment of the suture as well as frangible container co-packaged in a unified package, with a portion of suture disposed in the porous sponge. Frangible container is positioned inside the sponge.
FIG. 10B shows a schematic side cross-sectional view of an embodiment of the suture as well as frangible container co-packaged in a unified package, with a portion of suture disposed in porous sponge. Frangible container is positioned proximal to the sponge.
FIG. 11A shows a schematic side cross-sectional view of an embodiment of the suture as well as frangible container co-packaged in a unified package, with the suture not disposed in porous sponge in advance. Frangible container is positioned inside the sponge.
FIG. 11B shows a schematic side cross-sectional view of an embodiment of the suture as well as frangible container co-packaged in a unified package, with the suture not disposed in porous sponge in advance. Frangible container is positioned proximal to the sponge.
FIG. 12A shows a schematic side cross-sectional view of an embodiment of the suture as well as the sponge co-packaged in a unified package, with a portion of the suture disposed in porous sponge in advance. Also shown a dispenser container containing either a beneficial medicant for application to sponge, or a solvent for application to sponge impregnated with dry medicant.
FIG. 12B shows a schematic side cross-sectional view of an embodiment of the suture as well as the sponge co-packaged in a unified package, with the suture not disposed in porous sponge in advance. Also shown a dispenser container containing either a beneficial medicant for application to sponge, or a solvent for application to sponge impregnated with dry medicant.

### DETAILED DESCRIPTION

Briefly, the inventive suture system advantageously enables delivery of a beneficial agent or a medicant, or an additional or increased amount of a medicant, such as anti-bacterial, anti-microbial, anti-infection, any drug, such as cancer treatment drug, analgesic, hemostatic material, healing promoting agent, antibody, or similar, and combinations thereof, to the sutured wound or tissue, using any surgical suture (monofilament, braid, absorbable, non-absorbable, etc.), without or with only relatively minor changes in the material, coatings, and mechanical properties of the surgical suture as the tissue closure and fastener element. Most preferably, there are no changes to existing and approved for use sutures and needles prior to application of the medicants to the suture according to the present invention. The medicant can also comprise a swellable material, and/or any excipients and additives needed to enhance action or facilitate formulation. The term "medicant" here denotes any beneficial agent, without limitation, including medically useful, handling useful, imaging useful, swellable, tissue interaction and or tissue bonding beneficial, dye compound, sensing, antimicrobial, healing, bioactive, etc., beneficial agents, and combinations thereof. Further, the term "medicant" can also refer to adhesives, sealants, polymeric swellers, thickening agents (viscosity increasing), chemical indicators, biological indicators, UV coatings, coatings visible at specific light wavelengths, controlled release agents, suture knot strength enhancing compound or suture knot locking adhesives, and combinations thereof.

According to some embodiments, there is provided a frangible and or pierceable container containing a beneficial medicant in a solution, which is brought into contact with the surgical suture immediately before suture installation into the tissue. According to alternative embodiments, there is provided a frangible container containing a solvent which is released into a sponge media containing a beneficial medicant, the solvent solubilizing the medicant, which is then brought into contact with the suture immediately before suture installation into the tissue. According to some embodiments, a separate non-frangible container with medicant solution or with a solvent is provided and used to apply to a sponge media for transfer to suture. Transfer of medicant to suture occurs directly in the pierceable container or via sponge media wetted with the solution, with suture picking up some medicant solution and/or being coated by the medicant solution on contact with such solution in the pierceable container or in the sponge.

The suture is contacted with the beneficial medicant within seconds to minutes prior to installation into the tissue, such as about 5, 10, 30, 50 seconds prior to installation, or such as about 1, 3, 5, 10, 30, 60, 120 minutes prior to installation. Preferably, the suture is contacted with the beneficial medicant after the sterilization treatment of the suture and of the medicant was performed when the suture and the medicant are not in physical contact, i.e. sterilization of sutures and medicants is performed before the medicant is coated onto and/or impregnated into, the suture. The suture is contacted with the beneficial medicant most preferably from about 5 seconds to about 30 minutes prior to installation into the tissue.

The beneficial medicant is picked up by the suture and is carried into the tissue for subsequent action on the interface between the suture and tissue and optional release into the surrounding tissue for medically beneficial effects such as anti-infective, anti-bacterial, pain relief, or any other medically beneficial effect or action of the medicant(s).

It is to be understood, that inventive sutures and methods may also be enabling longer shelf life by combining the medicant and the suture immediately prior to use, as compared to sutures pre-coated with medicants prior to sterilization and packaging.

### Suture and needle

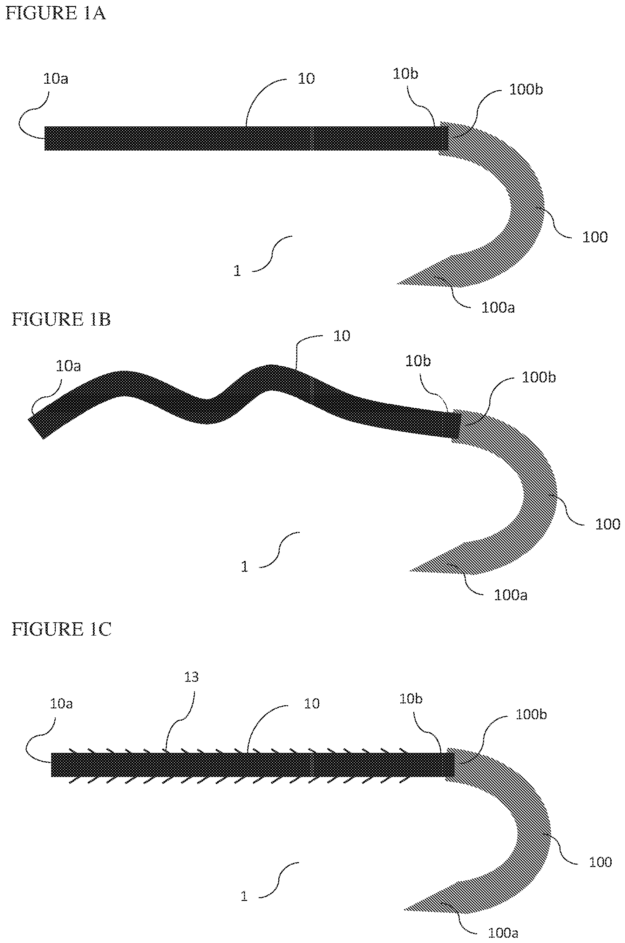

Referring to Figures 1A through 1C, showing schematic side cross-sectional views, showing suture 10 comprising an elongated flexible string, filament, yarn, bundle of filaments, braid, or similar, with suture 10 having connecting end 10b and opposing free end 10a, with suture 10 connecting end 10b connected to suture needle 100 at a needle connecting end 100b. Figure 1A, 1B shows non-barbed suture 10, and Figure 1C shows barbed or knotless suture having barbs 13. Needle 100 comprises an elongated straight or curved member having a sharpened end 100a opposite the needle connecting end 100b. Suture 10 connecting end 10b is typically inserted into an opening in needle connecting end 100b. As known in the art, suture 10 connecting end 10b can be fixated by swaging needle 100, using adhesives, etc. Free end 10a can optionally have a stop or tab (not shown) that fixates free end 10a in tissue. Suture 10 with attached needle 100 forms + 1.

### Pierceable container with medicant: embodiments, methods, and kits

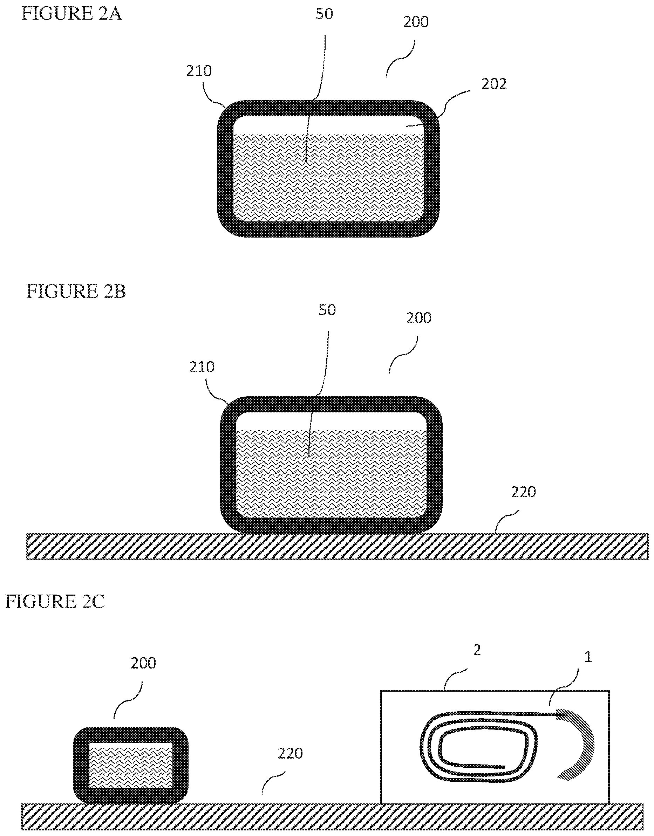

Referring to Figure 2A, in one embodiment, pierceable container 200 is formed by pierceable wall 210 and contains beneficial medicant as a liquid solution 50, such as a solution in a solvent, i.e. water, saline, ethanol, or similar. Pierceable wall 210 is impenetrable to solution 50 and is easily pierceable by surgical needle 100. Materials include flexible and elastomeric polymetric materials, fluoroelastomers, silicone, rubber, synthetic rubber, neoprene, etc. Container 200 can be filled with solution 50 with no remaining space inside, i.e. no gas phase (not shown), or it can be filled with a gas phase 202 therein as shown in Fig. 2A.

Referring to Figure 2B, pierceable container 200 is mounted on a solid, preferably planar support 220, thus fixating container 200 and simplifying piercing of container 200 by needle 100.

Referring to Figure 2C, connected suture 1 is enclosed in suture packaging box 2. As shown, both pierceable container 200 and connected suture 1 enclosed in suture packaging box 2 are mounted on solid support 220, thus fixating container 200 in proximity to connected suture 1 and forming a kit for applying beneficial medicant to suture 10. In alternative embodiments (not shown), pierceable container 200 and connected suture 1 are not enclosed in the same suture packaging box 2, but are supplied separately.

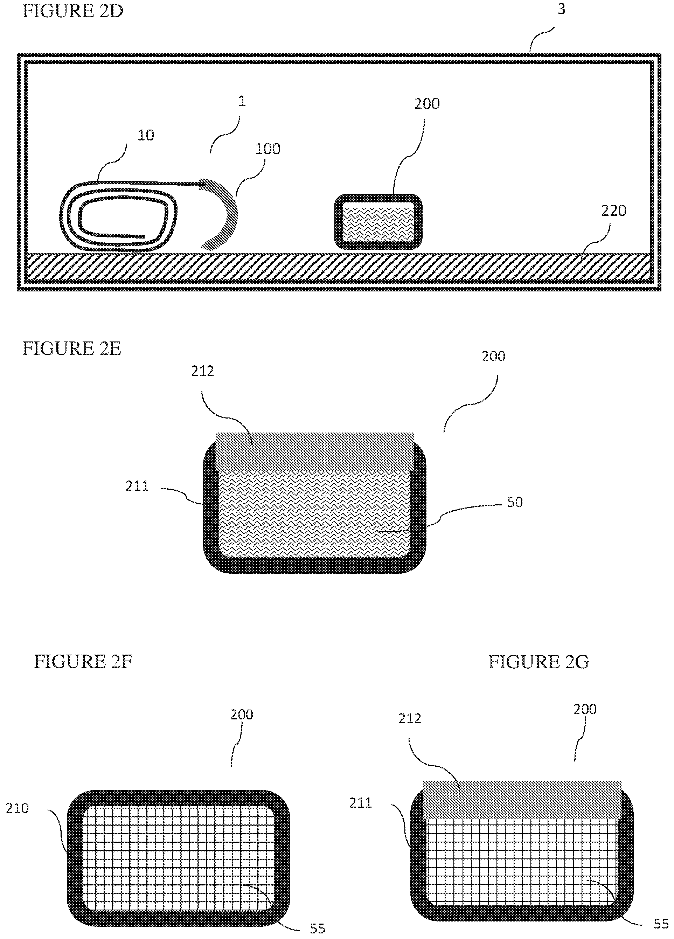

Figure 2D schematically shows an embodiment with connected suture 1 and pierceable container 200 mounted on solid support 220 and further enclosed in a unified package 3.

In some embodiments, referring to Figure 2E, pierceable container 200 containing beneficial medicant as a liquid solution 50 is formed by generally non-pierceable wall 211 and closed with a pierceable septum 212 that is easily pierceable by surgical needle 100. Wall 211 and septum 212 are impenetrable to liquid solution 50. Septum materials include flexible and elastomeric polymetric materials, fluoroelastomers, silicone, rubber, synthetic rubber, neoprene, etc. Container 200 can be filled with solution 50 with no remaining gas phase or space inside (as shown), or container 200 can be filled with gas phase therein (not shown).

In some embodiments, referring to Figure 2F, pierceable container 200 is formed by pierceable wall 210 and contains beneficial medicant as a liquid solution distributed in a sponge 55. Container 200 is at least partially filled by sponge 55 that is wetted with beneficial medicant as a liquid solution in a solvent. Pierceable wall 210 is impenetrable to medicant solution but is easily pierceable by surgical needle 100.

In some embodiments, referring to Figure 2G, pierceable container 200 is formed by generally non-pierceable wall 211 and closed with a pierceable septum 212 that is easily pierceable by surgical needle 100. Pierceable container 200 contains beneficial medicant as a liquid solution distributed in sponge 55. Container 200 is at least partially filled with sponge 55 that is wetted with beneficial medicant as a liquid solution in a solvent.

Sponge 55 is any porous, inert material that is capable of accepting and wicking fluids and distributing fluids throughout the sponge. At least some of the pores are interconnected, such as in predominantly open cell foams and sponges, enabling fluids to rapidly move and distribute through sponge 55. Sponge 55 is easily penetrable and pierceable by needle 100.

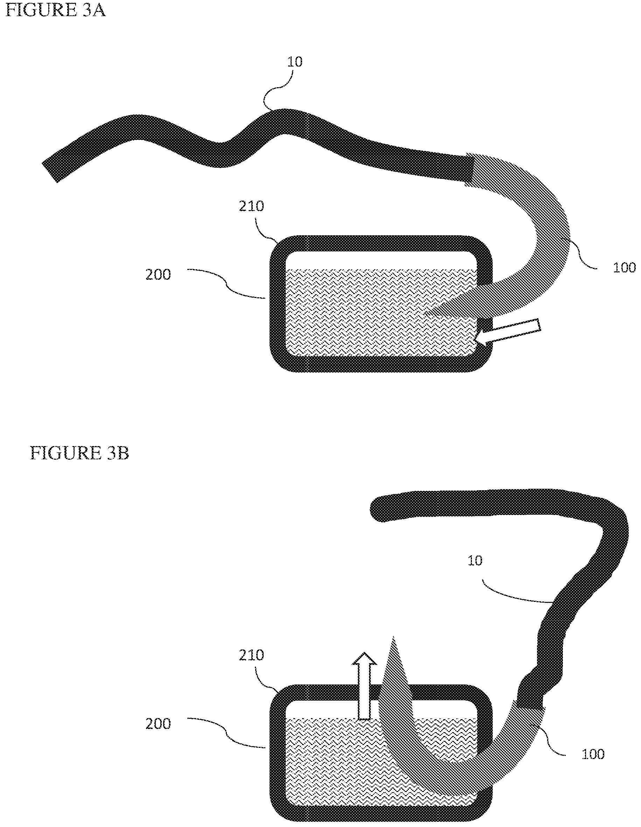

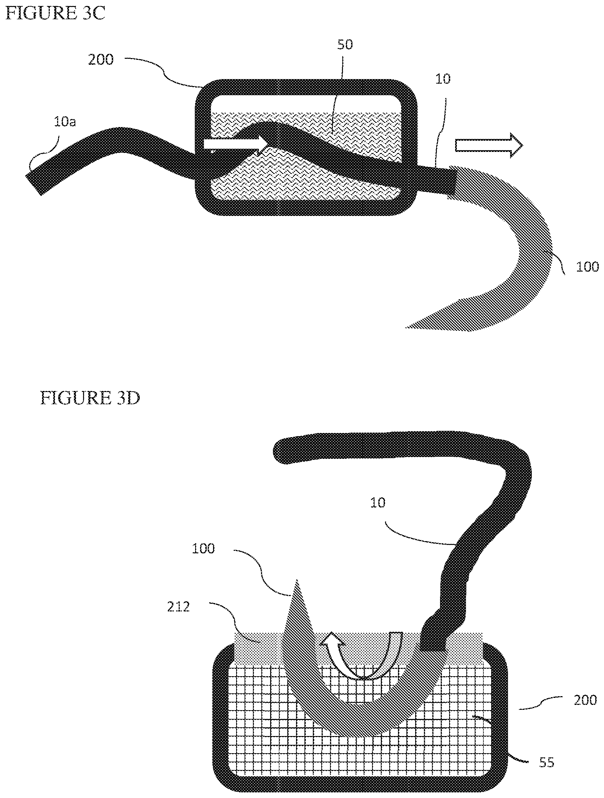

In use, and referring to Figure 3A, a health practitioner uses needle 100 to pierce wall 210 of container 200 from outside, or alternatively piercing septum 212 (not shown in Figure 3A), entering container 200 with the needle 100, and then, as shown in Figure 3B, exit container 200 with needle 100 in a different spot, piercing container wall 210 from inside, removing needle 100 from container 200 all the while pulling suture 10 behind needle 100 through container 200, as seen in Figure 3C. Arrows in Figures 3A-C show directionality of movement or pulling of needle 100 and suture 10 through container 200.

As can be appreciated, suture 10 is passing through container 200, contacting solution of beneficial medicant 50 therein, and exits container 200 after such contact with solution 50. Continuing pulling suture 10 through container 200, eventually all suture 10 passes though container 200 and exits container 200 with free end 10a exiting last. In this embodiment, free end 10a has no optional stop or tab for fixating free end 10a in tissue. Advantageously, as suture 10 is exiting container wall 210 through hole pierced by needle 100, excess medicant solution 50 is wiped off suture 10 via interaction with wall 210 as wall 210 closes around suture 10.

Advantageously, after exiting container 200, suture 10 has been contacted with medicant solution 50 and coated and or wetted with medicant solution 50.

Figure 3D illustrates another embodiment showing container 200 closed by pierceable septum 212 and having inside sponge 55 that is wetted with beneficial medicant. As shown, needle pierceable septum 212 closing container 200 and sponge 55 inside container 200 are penetrated by needle 100, with suture 10 pulled behind needle 100, operating similarly to the above embodiments. Needle 100 entering and exiting through septum 212 and or needle 100 entering and exiting sponge 55 inside container 200, with suture 10 following needle 100 and entering and exiting septum 212 and or entering and exiting sponge 55. As suture 10 is exiting septum 212 through hole pierced by needle 100, excess medicant solution 50 is wiped off suture 10 via interaction with septum 212 as septum 212 closes around suture 10.

In all embodiments above, pulling suture 10 through container 200, eventually all suture 10 passes though container 200 and exits container 200 with free end 10a exiting last. Advantageously, after exiting container 200, suture 10 has been wetted and or coated with medicant solution 50.

Connected suture 1 can be used immediately for installation into the tissue, such as within 1, 5, 10, 30, 60, 120 seconds after exiting container 200. Alternatively or additionally, health practitioner can wait to allow solvent at least partially evaporate from suture 10, in which case connected suture 1 can be used for installation into the tissue after partial or full evaporation of the solvent, leaving beneficial medicant on or in suture 10 and none or only a portion of solvent on or in suture 10. In this embodiment, connected suture 1 can be used for installation into the tissue within 1,2,3, 5, 10, 20, 30, 60, 120 minutes after exiting container 200. Advantageously, suture 10 contains beneficial medicant of its surface and or within suture pores, interstices, under barbs, between yarns or filaments forming suture 10 braids, or anywhere on or within suture 10 or suture 10 coatings, if any. The rate of pulling of suture 10 through container 200 is from about 5 to about 60 seconds to pull all suture 10 through, such as about 5, 10, 20, 30, 45, 60 seconds. In some embodiments, suture 10 is pulled at a rate of about 1, 2, 3, 4, 5 cm per second.

The described system, kit, and process show how a suture coated and or impregnated with beneficial medicant is formed prior to installation into tissue. The beneficial medicant is picked up by the suture and is carried into the tissue for subsequent action on the interface between the suture and tissue and optional release into the surrounding tissue for medically beneficial effects such as anti-infective, anti-bacterial action. In some embodiments (not shown), there are provided two pierceable containers 200 containing different beneficial agents or medicants as a liquid solution 50, potentially reactive with each other. In case the different beneficial medicants are reactive, the system is referred to as binary or multi-component coating system, with suture contacted each of the two different beneficial agents or medicants sequentially, causing the binary system components to react on the suture. The components of such binary system may include (i) monomers and initiators or catalysts of polymerization of said monomers, to form polymerized coating on the suture; (ii) acidic and alkaline compounds, with such compounds reacting resulting in a salt formation or in gas formation, such as N₂O₂ trapped in a viscous coating for slow release; (iii) coatings, hygroscopic compounds, or similar, with the coatings protecting hygroscopic compounds during sterilization until use. In all cases, the components of the binary coating system are brought together on the suture immediately before the using of the suture and can beneficially react with each other prior to suture implantation, during the suture implantation, or immediately after the suture implantation.

### Frangible container with medicant; wicking sponge: embodiments, methods, and kits

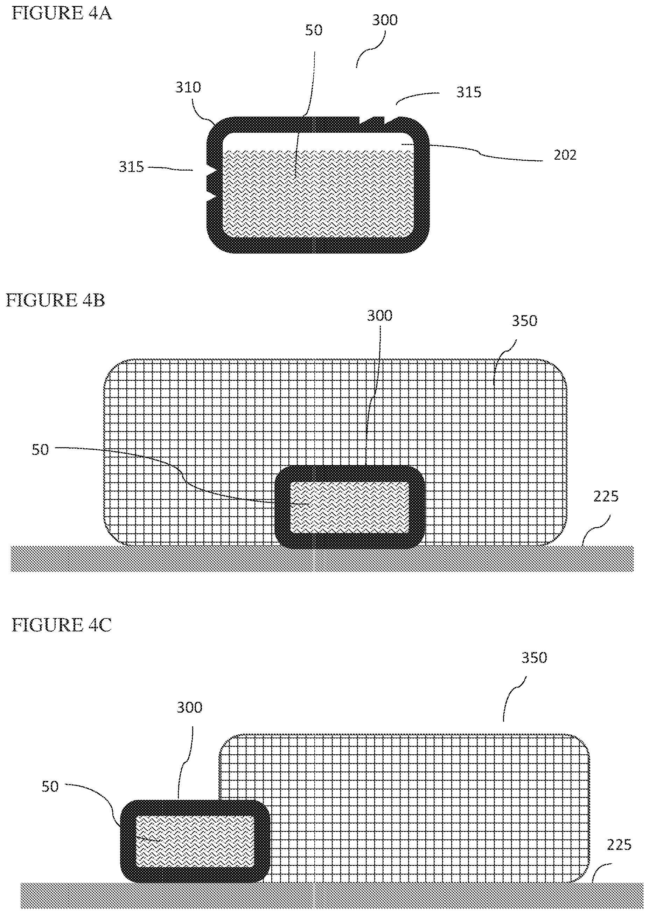

Referring to Figure 4A, in one embodiment, frangible container 300 is formed by frangible, easily breakable wall 310 and contains beneficial medicant as a liquid solution 50, such as a solution in a solvent, i.e. water, saline, ethanol, or similar. Frangible wall 310 is impenetrable to solution 50 and can be broken, cracked, shattered, fragmented, or crushed by applying pressure onto frangible container 300 with a blunt tool, such as a handle of a surgical instrument, e.g. scalpel, dissector, etc., or by a gloved finger of practitioner. Materials for wall 310 include non-flexible and non-elastomeric or partially flexible and partially elastomeric polymetric materials, glass, gelatin, etc. Optionally, frangible wall 310 has one or more indents or cut-outs 315, where some material of wall 310 is removed making it more susceptible to fragment and crack under pressure, releasing liquid solution 50. Frangible container 300 can be filled with solution 50 with no remaining space inside, i.e. no gas phase (not shown), or it can be filled with a gas phase 202 therein as shown in Fig. 4A.

As shown in Figure 4B, in some embodiments, frangible container 300 is mounted inside porous transfer sponge or foam 350, with both frangible container 300 and porous sponge 350 mounted on a solid support 225, optionally forming a kit. Sponge 350 is any porous, inert material that is capable of accepting and wicking fluids and distributing fluids throughout the sponge. At least some of the pores are interconnected, such as in predominantly open cell foams and sponges, enabling fluids to rapidly move through sponge. In some embodiments, sponge 350 is swelling upon contact with liquids. Frangible container 300 can be filled with solution 50 with no remaining space inside, i.e. no gas phase as shown in Fig. 4B, or with gas phase therein (not shown).

As shown in Figure 4C, frangible container 300 can be mounted not inside, but in contact with porous sponge 350, or partially inside sponge 350, whereby both frangible container 300 and porous sponge 350 are mounted on a solid support 225.

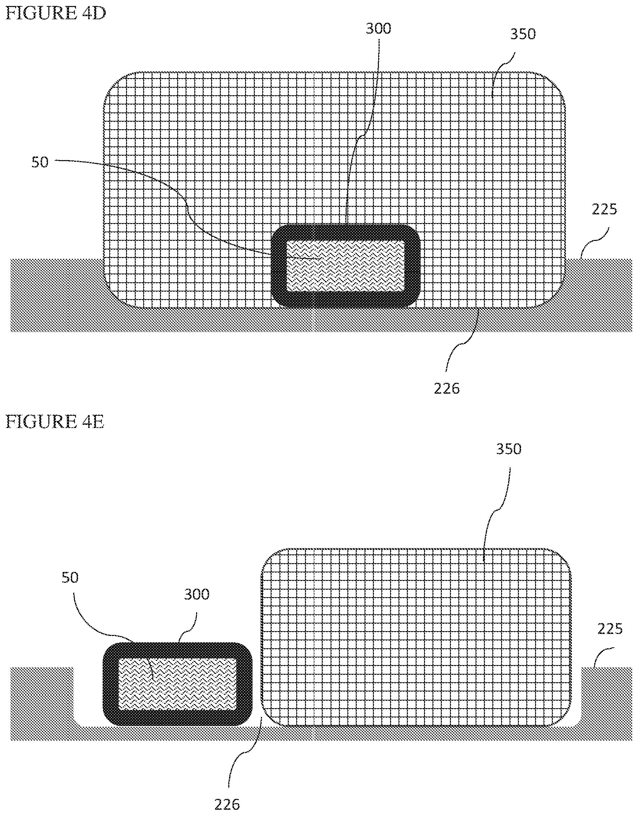

As shown in Figure 4D, solid support 225 can have an indentation or cut-out or basin 226 for capturing liquid solution 50 released upon breaking frangible container 300, with frangible container 300 mounted within basin 226 and inside porous sponge 350.

As shown in Figure 4E, solid support 225 can have an indentation or cut-out or basin 226 for capturing liquid solution 50 released upon breaking frangible container 300, with frangible container 300 mounted within or near basin 226 but not inside porous sponge 350. Frangible container 300 can be mounted near porous sponge 350 and not in contact (as shown) or contacting porous sponge 350 (not shown in Figure 4E). Sponge 350 is preferably at least partially disposed within basin 226 as shown.

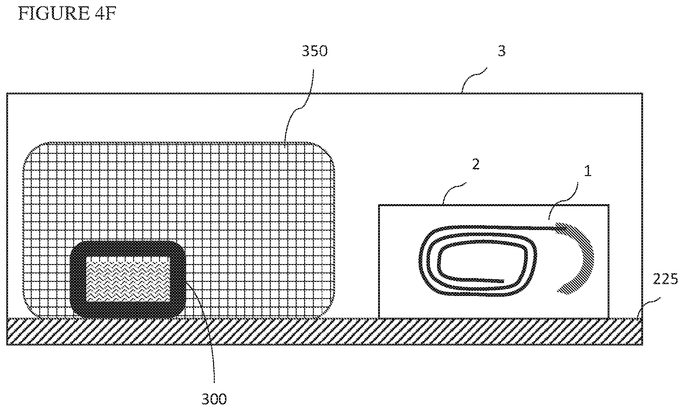

In some embodiments, as shown in Figure 4F, suture 1 is enclosed in suture packaging box 2. As shown, frangible container 300, porous sponge 350, and suture 1 enclosed in suture packaging box 2 are mounted on solid support 225, thus fixating container 300 in proximity to suture 1 and forming a kit for applying beneficial medicant to suture 10. suture 1, frangible container 300, porous sponge 350 can all be optionally enclosed in a unified package 3. In alternative embodiments (not shown) frangible container 300, porous sponge 350, and suture 1 are not all enclosed in suture packaging box 2, with frangible container 300 packaged separately. In some embodiments (not shown) frangible container 300 is not enclosed in unified package 3.

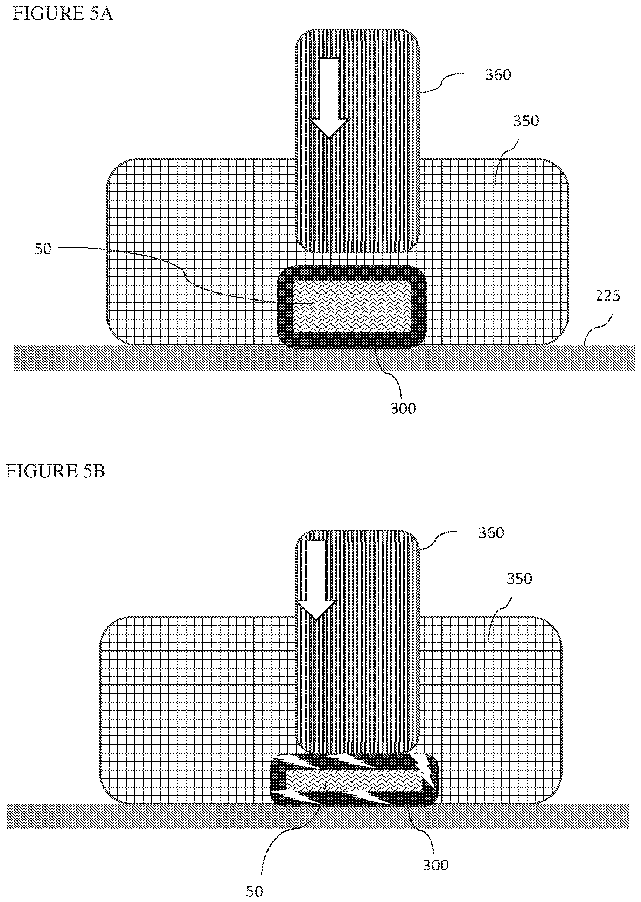

In use, and referring to Figure 5A, health practitioner applies a sterile blunt tool 360 through compliant, compressible, elastic porous sponge 350 to frangible container 300. The directionality of movement is indicated by the arrow.

Referring to Figure 5B, as the blunt tool 360 reaches frangible container 300 by compressing sponge 350. Container 300 is deformed, causing it to crack, open, and release liquid 50 into surrounding sponge 350. Tool 360 is then withdrawn and removed.

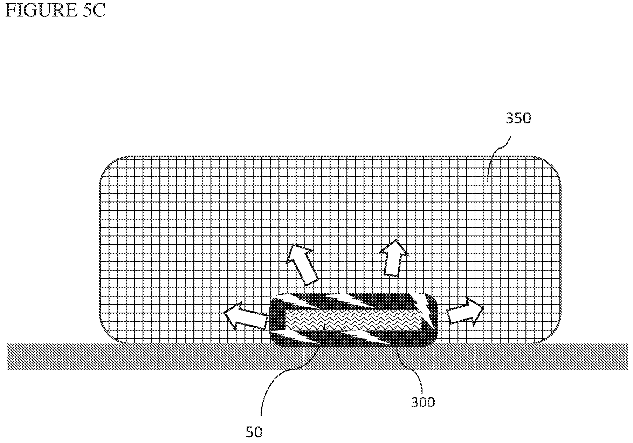

Referring to Figure 5C, following crushing of frangible container 300, liquid solution 50 is leaking outside of cracked container 300 and is rapidly wicked into sponge 350, with solution 50 movement directionality schematically shown by arrows. Sponge 350 picking up solution 50 and being wetted by solution 50, forming sponge 350 infused with solution 50.

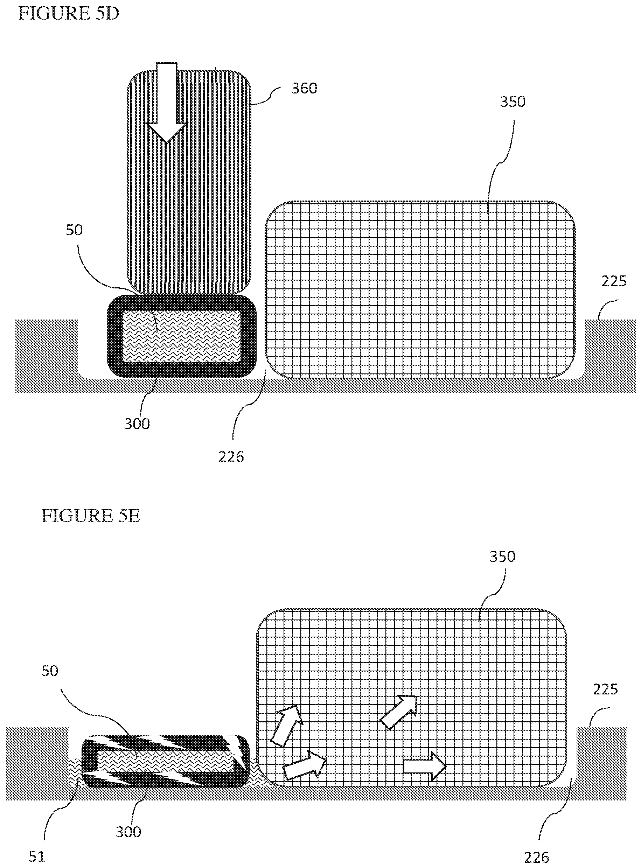

Similar methods are applicable to other embodiments and arrangements of frangible container 300, sponge 350, and support 225. In one embodiment, as shown in Figure 5D, blunt tool 360 is applied directly to container 300, which is positioned outside of sponge 350. As container 300 cracks, it releases liquid solution 50 into basin 226, with the released solution shown as 51 in basin 226 in Figure 5E. Solution 51 is then wicked into sponge 350 with solution 51 movement directionality schematically shown by arrows in Figure 5E. Sponge 350 is wicking solution 50 and is being wetted by solution 50, forming sponge 350 infused with solution 50.

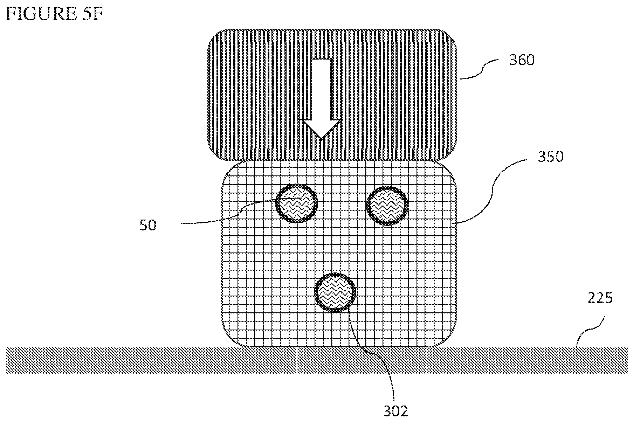

As shown in Figure 5F, in an alternative embodiment, frangible container is represented by a plurality of frangible, easily breakable capsules or microcapsules 302 containing beneficial medicant as a liquid solution 50. Capsules 302 are all positioned within sponge 350 and can be broken, cracked, shattered, fragmented, or crushed by applying pressure with a blunt tool 360 onto compressible and elastic sponge 350.

After sponge 350 was infused with solution 50 from frangible container 300, as shown above, suture 10 is coated with solution 50 by pulling suture 10 through sponge 350.

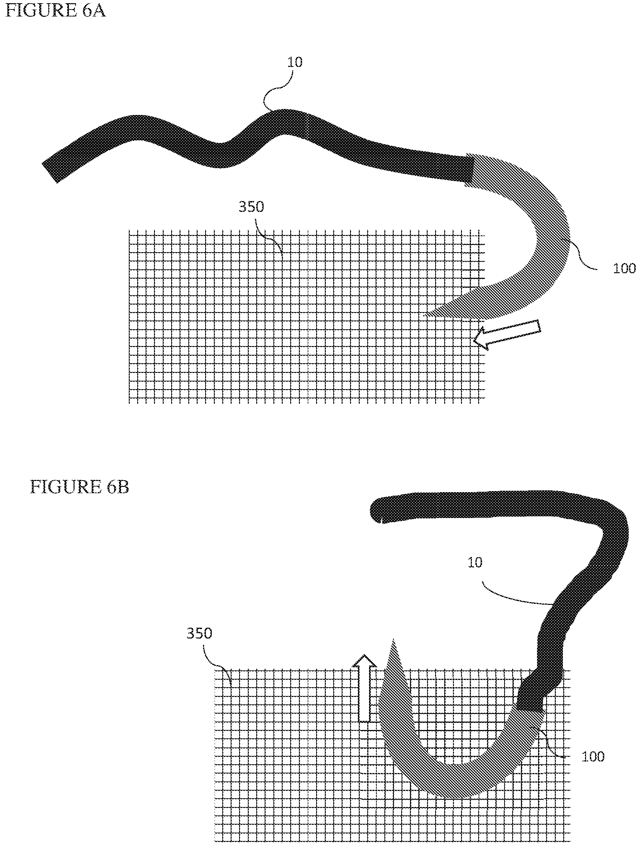

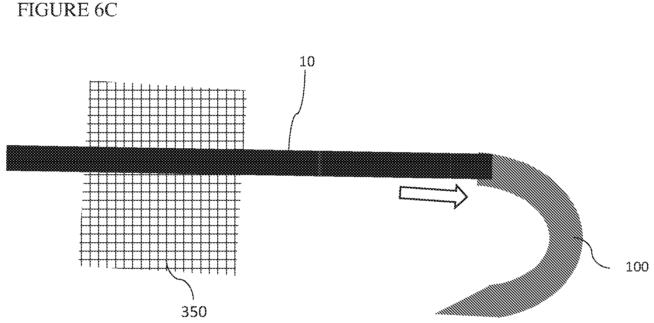

As shown in Figures 6A, 6B, 6C in one embodiment, needle 100 is piercing sponge 350 from outside, entering sponge 350 and then exiting sponge 350, while pulling suture 10 behind needle 100 through sponge 350. Arrows in Figures 6A-C show directionality of movement or pulling of needle 100 and suture 10 through sponge 350.

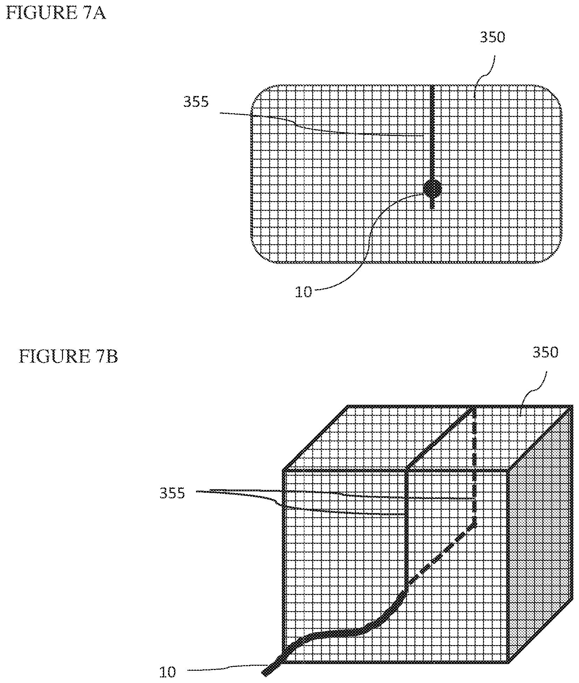

As shown in Figures 7A, showing schematic side view (with suture 10 shown perpendicular to the plane of view), and 7B, 7C, showing schematic perspective views, in another embodiment, suture 10 is positioned in the sponge 350, inside an aperture in sponge 350 (not shown) or preferably in a slit 355 formed in sponge 350. Slit 355 is a linear cut or slit made through a portion of sponge 350, formed so as to enable positioning suture 10 inside sponge 350 by placement of suture 10 in the slit 355. Sponge 350 closes around suture 10 inside slit 355 and suture 10 can be pulled out of sponge 350 infused with solution 50, maintaining contact of suture 10 with sponge 350. After sponge 350 was infused with solution 50 from frangible container 300, as shown supra, suture 10 is coated with solution 50 by pulling suture 10 through sponge 350.

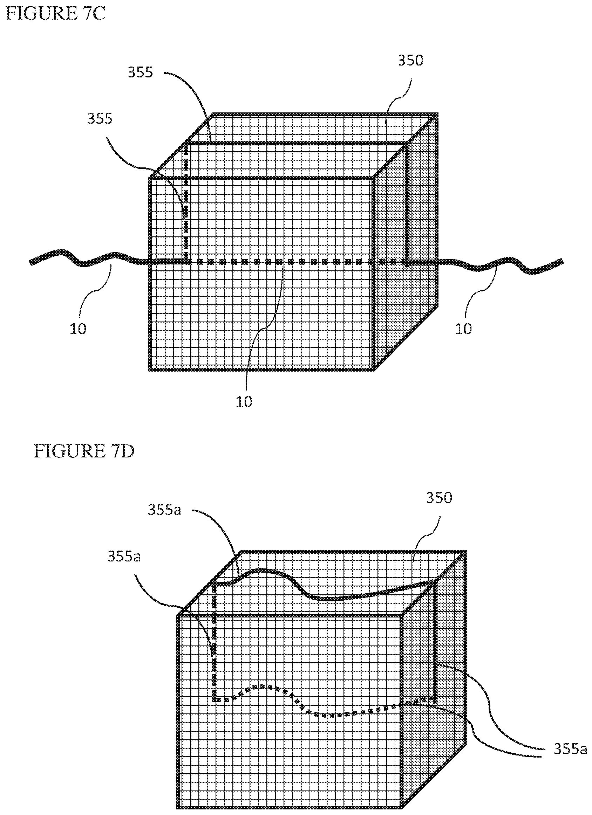

Figure 7D presents a schematic perspective view of another embodiment of sponge 350, with suture 10 not shown, whereby slit 355a is a non-linear cut or slit made through a portion of sponge 350, with non-linear or undulating slit advantageously providing for longer path or tortuous path of suture 10 through sponge 350, thus increasing contact of suture 10 with sponge 350 and medicant solution 50 and/or longer residence time of suture 10 inside sponge 350.

In one embodiment, suture 10 is positioned in slit 355 prior to breaking frangible container 300, i.e. prior to infusing sponge 350 with solution 50. In another embodiment, suture 10 is positioned in slit 355 after breaking frangible container 300, i.e. after infusing sponge 350 with solution 50. Advantageously, as suture 10 is passing through sponge 350, it is being coated with beneficial medicant, with excess medicant solution 50 is wiped off suture 10 via interaction with sponge 350. Advantageously, after exiting sponge 350, suture 10 has been wetted with solution 50.

Connected suture 1 can be used immediately for installation into the tissue, such as within 1, 5, 10, 30, 60, 120, 180 seconds after exiting sponge 350. Alternatively or additionally, health practitioner can wait to allow solvent at least partially evaporate from suture 10, in which case connected suture 1 can be used for installation into the tissue after partial or full evaporation of the solvent, leaving mostly or only beneficial medicant on or in suture 10, such as within 1,2,3, 5, 10, 20, 30, 60 minutes after exiting sponge 350. Advantageously, suture 10 contains beneficial medicant of its surface and or within suture pores, interstices, under barbs, between yarns or filaments forming suture 10 braids, or anywhere on or within suture 10 or suture 10 coatings, if any. The rate of pulling of suture 10 through sponge 350 is from about 5 to about 60 seconds to pull all suture 10 through, such as about 5, 10, 20, 30, 45, 60 seconds. In some embodiments, suture 10 is pulled at a rate of about 1, 2, 3, 4, 5 cm per second.

The described system, kit, and process show how a suture coated and or impregnated with beneficial medicant is formed prior to installation into tissue. The beneficial medicant is picked up by the suture and is carried into the tissue for subsequent action on the interface between the suture and tissue and optional release into the surrounding tissue for medically beneficial effects such as anti-infective, anti-bacterial action.

### Frangible container with solvent; wicking sponge with dry medicant: embodiments, methods, and kits

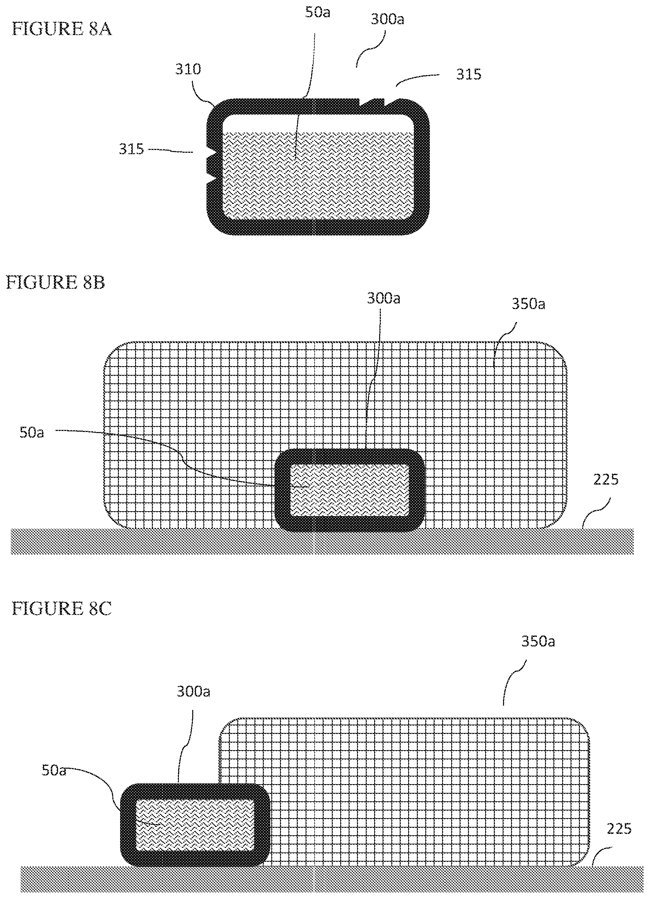

Referring to Figure 8A, in an alternative embodiment, frangible container 300a is formed by frangible, easily breakable wall 310 and contains solvent 50a, such as water, saline, ethanol, or similar, but no medicant. Frangible wall 310 is impenetrable to solvent 50a and can be broken, cracked, shattered, fragmented, or crushed by applying pressure with a blunt tool, such as a handle of a surgical instrument, e.g. scalpel, dissector, etc., or by a gloved finger of practitioner. Materials include non-flexible and non-elastomeric polymetric materials, glass, etc. Optionally, frangible wall 310 has one or more indents or cut-outs 315, where some material of wall 310 is removed making it more susceptible to fragment and crack under pressure, releasing solvent 50a.

As shown in Figure 8B, in one embodiment, frangible container 300a is preferably mounted inside porous sponge or foam 350a, with both frangible container 300a and porous sponge 350a mounted on a solid support 225, optionally forming a kit. Sponge 350a is any porous, inert material that is capable of accepting and wicking fluids and distributing fluids throughout the sponge. At least some of the pores are interconnected, such as in predominantly open cell foams and sponges, enabling fluids to rapidly move through sponge. In some embodiments, sponge 350 is swelling upon contact with liquids.

### Sponge 350a coated or impregnated with dry beneficial agent or medicant

Sponge 350a is different from sponge 350 shown in embodiments of Figures 4-7 in one differentiating aspect. Specifically, sponge 350a is coated throughout or impregnated with dry beneficial agent or medicant, at least partially soluble in solvent 50a. Dry medicant is rapidly soluble in solvent 50a, such as soluble on contact such as soluble within 2, 5, 10, 30, 60, 120, 180, 240 seconds, fully soluble, or at least partially soluble whereby at least 10, 25, 50, 75% of medicant is soluble within the times shown supra.

Sponge 350a is formed by any known techniques to impregnate it with dry medicant that is rapidly soluble in solvent 50a. In one method, sponge 350 wetted with a solution of medicant in any appropriate solvent, fully saturated with such solution, and then dried, such as dried by (i) allowing solvent to evaporate, (ii) dried under vacuum, (iii) freeze-dried, (iv) dried by heating, or any similar method or combinations thereof. Sponge 350a contains from .0001% to 20% of dry medicant by weight, such as 0.0001, 0.001, 0.001, 0.01, 0.1, 1, 2, 3, 4, 5, 7, 10, 20% of medicant by weight.

As shown in Figure 8C, in one embodiment, and similarly to embodiment shown in Figure 4C, frangible container 300a can be mounted not inside, but in contact with porous sponge 350a, whereby both frangible container 300a and porous sponge 350a are mounted on a solid support 225.

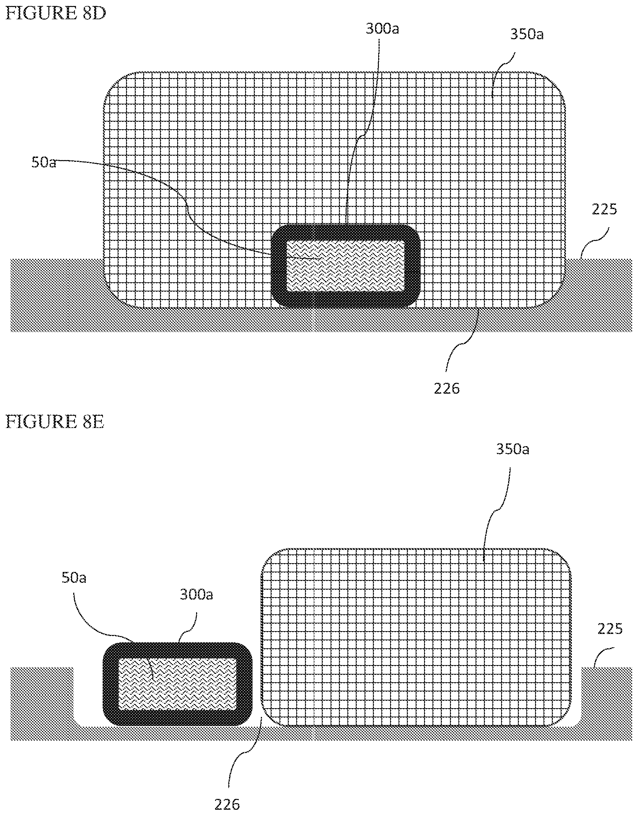

As shown in Figure 8D, in one embodiment, and similarly to embodiment shown in Figure 4D, solid support 225 can have an indentation or cut-out or basin 226 for capturing solvent 50a released upon breaking frangible container 300a, with frangible container 300a and sponge 350a at least partially mounted within basin 226 and frangible container 300a mounted inside porous sponge 350a.

As shown in Figure 8E, in one embodiment, and similarly to embodiment shown in Figure 4E, solid support 225 can have an indentation or cut-out or basin 226 for capturing solvent 50a released upon breaking frangible container 300a, with frangible container 300a mounted within or near basin 226 but not inside porous sponge 350a. Frangible container 300a can be mounted near porous sponge 350a and not in contact (as shown) or contacting porous sponge 350a (not shown in Figure 8E). Frangible container 300a and sponge 350a are at least partially mounted within basin 226.

Similarly to embodiments shown in Figure 4F, connected suture 1 can be enclosed in suture packaging box 2, and frangible container 300a, porous sponge 350a, and connected suture 1 enclosed in suture packaging box 2 can be mounted on solid support 225, thus fixating container 300a in proximity to connected suture 1 and forming a kit for applying beneficial medicant to suture 10.

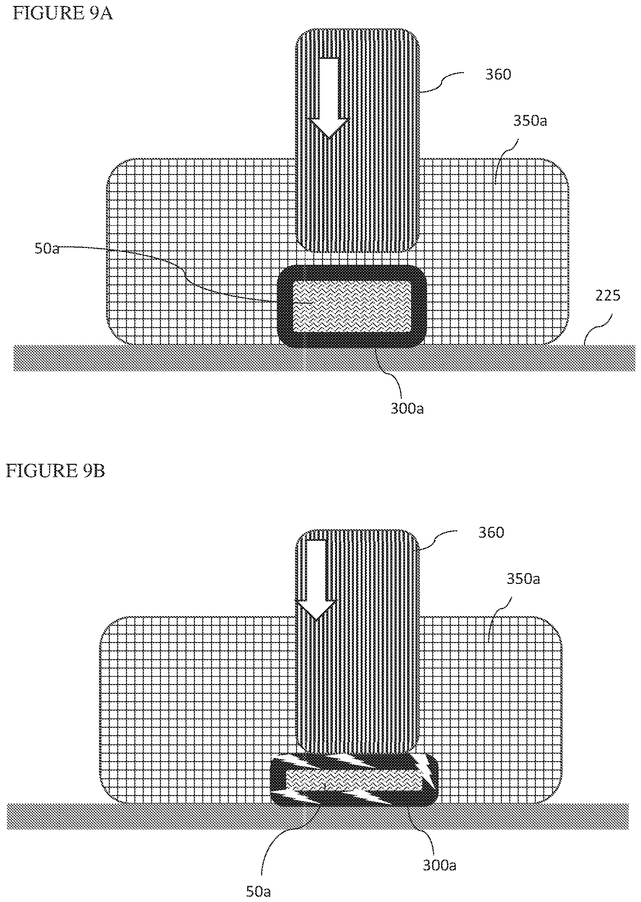

As shown in Figure 9A, in use, in one embodiment, and similarly to embodiment shown Figure 5A, health practitioner applies a sterile blunt tool 360 through compliant, elastic porous sponge 350a to frangible container 300a.

Referring to Figure 9B, as the blunt tool 360 reaches frangible container 300a. Container 300a is deformed, causing it to crack, open, and release solvent 50a into surrounding sponge 350a. Tool 360 is then withdrawn and removed.

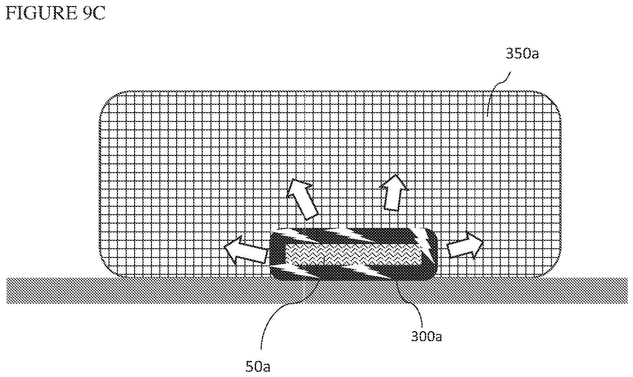

Referring to Figure 9C, following crushing of frangible container 300a, solvent 50a is leaking outside of cracked container 300a and is rapidly wicked into sponge 350a, with solvent 50a movement directionality schematically shown by arrows. Sponge 350a picking up solvent 50a and being wetted by solvent 50a, forming sponge 350 infused with solvent 50a.

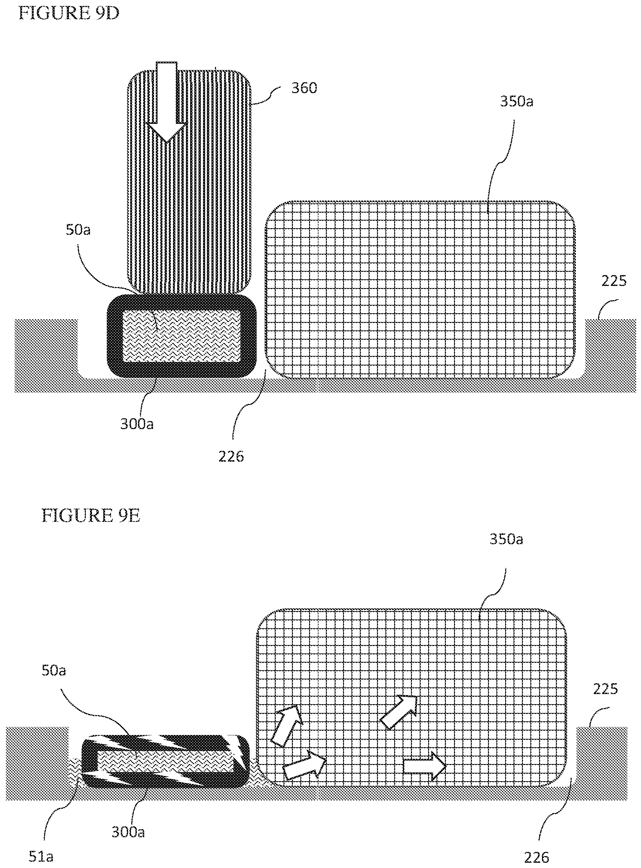

Similar methods are applicable to other embodiments and arrangements of frangible container 300a, sponge 350a, and support 225. In one embodiment, as shown in Figure 9D, blunt tool 360 is applied directly to container 300a. As container 300a cracks, it releases solvent 50a into basin 226, the solvent shown as 51a in basin 226 in Figure 9E. Solvent 50a is then wicked into sponge 350a with solvent 50a movement directionality schematically shown by arrows Figure 9E. Sponge 350a picking up solvent 50a and being wetted by solvent 50a, forming sponge 350a infused with solvent 50a.

In an alternative embodiment, similar to embodiments shown in Figure 5F, frangible container is represented by a plurality of frangible, easily breakable capsules or microcapsules 302 containing solvent 50a. Capsules 302 are all positioned within sponge 350a and can be broken, cracked, shattered, fragmented, or crushed by applying pressure with a blunt tool 360 onto sponge 350a.

During infusion of solvent 50a into the sponge, and after sponge 350a was infused with solvent 50a from frangible container 300a, as shown supra, dry medicant is dissolving or solubilizing in solvent 50a inside sponge 350a, forming sponge 350a infused with solution of medicant in solvent 50a.

Suture 10 is then coated with solution of medicant in solvent 50a by pulling suture 10 through sponge 350a. Similar to embodiments shown in Figures 6A, 6B, 6C, needle 100 is piercing sponge 350a from outside, entering sponge 350a and then exiting sponge 350a, while pulling suture 10 behind needle 100 through sponge 350a. Advantageously, as suture 10 is passing through sponge 350a, it is being coated with beneficial medicant, with excess medicant solution wiped off suture 10 via interaction with sponge 350a. Advantageously, after exiting sponge 350a, suture 10 has been wetted with solution of medicant in solvent 50a.

Similar to embodiments shown in Figure 7A, 7B, 7C, 7D, in another embodiment, suture 10 is positioned in the sponge 350a, inside an aperture in sponge 350a or preferably in a slit 355 formed in sponge 350a. Sponge 350a closes around suture 10 inside slit 355 and suture 10 can be pulled out of sponge 350a infused with solution of medicant in solvent 50a maintaining contact of suture 10 with sponge 350a. After sponge 350a was infused with solution of medicant in solvent 50a, as shown above, suture 10 is coated with solution of medicant in solvent 50a by pulling suture 10 through sponge 350a.

In some embodiments, slits 355, 355a are sized to provide easy positioning of suture 10 into slit 355, 355a in sponge 350, 350a. Upon wetting of sponges 350, 350a, sponges 350, 350a swell, closing slit 355, 355a around suture 10.

Connected suture 1 can be used immediately for installation into the tissue, such as within 1, 5, 10, 30, 60, 120 seconds after exiting sponge 350a. Alternatively or additionally, health practitioner can wait to allow solvent at least partially evaporate from suture 10, in which case connected suture 1 can be used for installation into the tissue after partial or full evaporation of the solvent, leaving only or mostly only beneficial medicant on or in suture 10, such as within 1,2,3, 5, 10, 20, 30, 60 minutes after exiting sponge 350a. Advantageously, suture 10 contains beneficial medicant of its surface and or within suture pores, interstices, under barbs, between yarns or filaments forming suture 10 braids, or anywhere on or within suture 10 or suture 10 coatings, if any. The rate of pulling of suture 10 through sponge 350a is from about 5 to about 60 seconds to pull all suture 10 through, such as about 5, 10, 20, 30, 45, 60 seconds. In some embodiments, suture 10 is pulled at a rate of about 1, 2, 3, 4, 5 cm per second.

The described system, kit, and process show how a suture coated and or impregnated with beneficial medicant is formed prior to installation into tissue. The beneficial medicant is picked up by the suture and is carried into the tissue for subsequent action on the interface between the suture and tissue and optional release into the surrounding tissue for medically beneficial effects such as anti-infective, anti-bacterial action.

### Packaging in unified containers

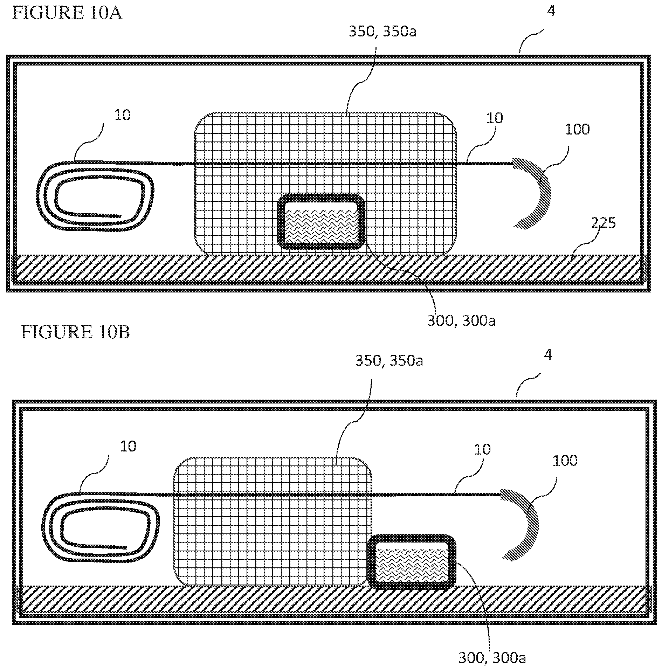

Referring to Figures 10A, 10B, in some embodiments, suture 10 attached to needle 100, sponge 350 or 350a, as well as frangible container 300 or 300a are all co-packaged in a unified package 4, with a portion of suture 10 proximal to needle 100 disposed in sponge 350 or 350a by positioning in a slit in sponge 350, 350a (slit not shown). In use, after opening unified package 4, frangible container 300 or 300a is crushed by a blunt tool as shown above in Figures 5A-5E, 9A-9E, thus releasing medicant solution from frangible container 300, or solvent from frangible container 300a into sponge 350, 350a. After sponge 350, 350a is wetted with medicant solution or solvent respectively, forming sponge 350, 350a wetted with medicant solution, suture 10 is pulled through sponge 350, 350a thus contacting suture 10 with medicant solution and wetting or coating suture 10 with medicant solution.

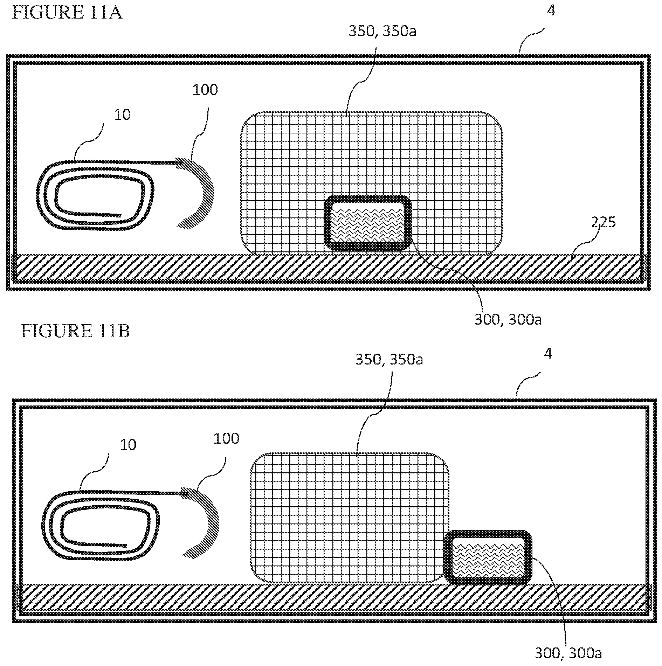

Referring to Figures 11A, 11B, in some embodiments, suture 10 attached to needle 100, sponge 350 or 350a, as well as frangible container 300 or 300a are all co-packaged in a unified package 4, with suture 10 not disposed in sponge 350 or 350a. In use, after opening unified package 4, frangible container 300 or 300a is crushed by a blunt tool as shown in Figures 4A-4E, 9A-9E, thus releasing medicant solution from frangible container 300, or solvent from frangible container 300a into sponge 350, 350a. After sponge 350, 350a is wetted with medicant solution or solvent respectively, forming sponge 350, 350a wetted with medicant solution, suture 10 is pulled through sponge 350, 350a thus contacting suture 10 with medicant solution and wetting or coating suture 10 with medicant solution. In this embodiment, pulling suture 10 through sponge 350, 350a is performed by any convenient technique. In one method, suture 10 can be placed into a slit in sponge 350, 350a (slit not shown in Figures 11A, 11B), similar to the embodiments of Figures 7A - 7D. In another method, similar to embodiments shown in Figures 6A, 6B, 6C, needle 100 is piercing sponge 350, 350a from outside, entering sponge 350, 350a and then exiting sponge 350, 350a, while pulling suture 10 behind needle 100 through sponge 350, 350a. Advantageously, as suture 10 is passing through sponge 350, 350a, it is being coated with beneficial medicant, with excess medicant solution wiped off suture 10 via interaction with sponge 350, 350a. Advantageously, after exiting sponge 350, 350a, suture 10 has been wetted or coated with solution of medicant.

### Embodiments with a separate container with medicant or solvent and no frangible container

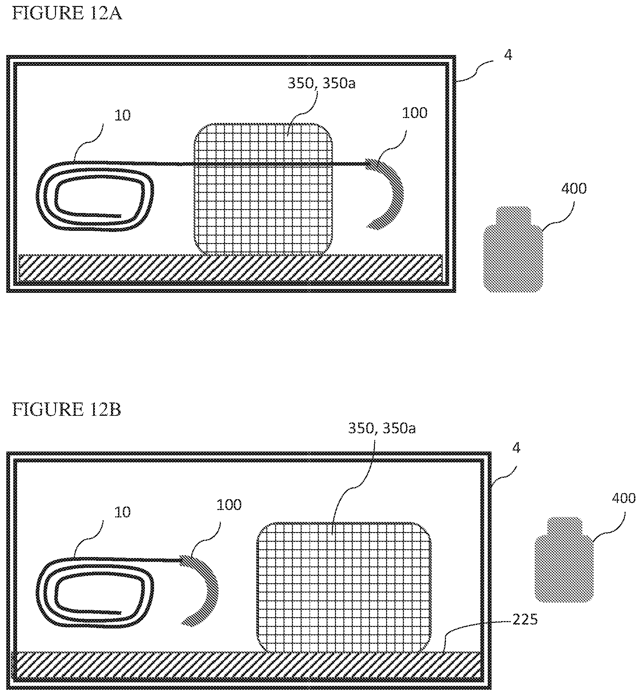

Referring to Figures 12A, 12B, in some embodiments, suture 10 attached to needle 100 as well as sponge 350 or 350a are packaged in a unified package 4. There is no frangible container.

As shown in Figure 12A, a portion of suture 10 proximal to needle 100 is disposed in sponge 350 or 350a by positioning in a slit in sponge 350, 350a (slit not shown). As shown in Figure 12B, in some embodiments, suture 10 is not disposed in sponge 350 or 350a.

Advantageously, there is also provided, as a part of a kit, or separately, a dispenser container 400 containing either a beneficial medicant for application to sponge 350, or a solvent for application to sponge 350a impregnated with dry medicant. In the embodiments shown in Figures 12A, 12B, in use, health practitioner opens dispenser container 400 and dispenses liquid containing beneficial medicant from container 400 onto sponge 350, thus forming sponge 350 wetted with solution of beneficial medicant. Alternatively, in use, health practitioner opens dispenser container 400 and dispenses solvent from container 400 onto sponge 350a that is impregnated with dry beneficial medicant, thus forming sponge 350a wetted with solution of beneficial medicant, upon dissolution of dry medicant contained in sponge 350a in solvent dispensed from dispenser 400. Suture 10 is then coated with medicant by being pulled through sponge 350, 350a wetted with beneficial medicant as described supra. In embodiments of Figure 12A, suture 10 is pulled through sponge 350, 350a directly. In embodiments of Figure 12B, in one method, suture 10 can be placed into a slit in sponge 350, 350a and then pulled through sponge 350, 350a. In another method, needle 100 is piercing sponge 350, 350a from outside, entering sponge 350, 350a and then exiting sponge 350, 350a, while pulling suture 10 behind needle 100 through sponge 350, 350a. In some embodiments, dispenser container 400 can be packaged or co-packaged in the unified package 4 or near the unified package 4, or alternatively separately from the unified package 4.

Advantageously, as suture 10 is passing through sponge 350, 350a, it is being coated with beneficial medicant, with excess medicant solution wiped off suture 10 via interaction with sponge 350, 350a. Advantageously, after exiting sponge 350, 350a, suture 10 has been wetted or coated with solution of medicant.

In some embodiments, there are more than one dispenser containers 400 containing different beneficial medicants or different concentrations of beneficial medicants for application to sponge 350. With different concentrations, the health practitioner can select which concentration is most appropriate to a particular patient and then apply that concentration of beneficial medicant solution to sponge 350 from appropriate dispenser container 400.

In some embodiments, two or more different drugs or agents are contained in two or more dispenser containers 400. The health practitioner can then apply either one of these agents to sponge 350 or can apply two or more different drugs or agents to the same sponge 350, forming a combination multi-agent medicant inside sponge 350.

### Alternative embodiments with two or more medicants located on different carriers

As shown above, more than one medicant can be present in frangible container 300 or in sponge 350a. In an alternative embodiment, frangible container 300 contains a first medicant in a solvent, and sponge 350a contains a dry second medicant that is soluble in the same solvent. Similar to the supra embodiments, upon crushing of frangible container 300, sponge 350a becomes infused with solutions of first medicant in the solvent and immediately also forming solution of the second medicant in the same solvent, this forming sponge 350a infused with solutions of two or more medicants in the same solvent.

In some embodiments, suture 10 is already coated with a first medicant(s), such as triclosan, CHG, silver, or similar. A second medicant in pierceable container 200 or frangible container 300 or sponge 350a containing dry medicant can be a) different medicant, including antimicrobial agent, or any other drug or combinations thereof, or b) the same medicant as the first medicant that is used to increase concentration of the first medicant on suture 10.

Advantageously, health practitioner can elect to add second medicant to the suture when needed due to the nature of surgery, co-morbidities, patient's health status, etc., or not to add the second medicant when not needed. Advantageously, two different medicants can be combined as needed, or in the case b) above, the same medicant can be increased in concentration on suture as needed.

In some embodiments, in the same package, there are provided two or more pierceable containers 200 or two or more frangible containers 300, or two or more dispensing containers 400, or two or more sponges 350a, containing different medicants or different concentrations of the same medicant for use as needed, with the health practitioner having a choice of which medicant to use by using appropriate pierceable container 200 or frangible container 300, or sponge 350a or dispensing container 400.

In some embodiments, a dual transfer sponge is provided, whereby a first medicant transfer sponge 350 or 350a is used to apply a first medicant to suture 10, and a second transfer sponge (not shown) is used apply a second medicant after exiting first transfer sponge 350 or 350a. In some embodiments the first medicant and the second medicant of such dual transfer sponge are potentially reactive with each other. In case the different beneficial medicants are reactive, the system is referred to as binary or multi-component coating system, with suture contacted each of the two different beneficial agents or medicants sequentially, causing the binary system components to react on the suture, as described above.

In some embodiments, a dual sponge is provided, whereby medicant transfer sponge 350 or 350a is used to apply medicant to suture 10, and a secondary wiping sponge (not shown) is used to run suture 10 through such sponge after exiting transfer sponge 350 or 350a so that excess medicant can be wiped off suture 10 within secondary sponge.

### STERILIZATION

Gamma, X-ray, e-beam , Ethylene Oxide (ETO), thermal, and any other sterilization technique can be utilized for sutures, medicants, and packages of the present invention. In some embodiments, the current invention enables use of the gamma sterilization and e-beam instead of ETO and use of a wide range of antimicrobial materials and medicants on the sutures. Any medicant can be used and the medicant need not be exposed to the high energy sterilization. The medicant sterilization can be performed by utilizing other methods, such as nano-filtration or aseptic filling. The medicant sterilization can be performed separately and by different technique vs sterilization of suture 10.

Suture and the medicant can be stored separately and combined immediately before use. Pierceable container or frangible container or sponge 350a can be sterilized together with suture 10, or advantageously separately from suture 10, and by a different technique, or a under different parameters (e.g. energy level, temperature) by the same technique.

In one embodiment, the system is subject to sterilization so that any component containing the medicant, i.e. pierceable container, frangible container, sponge 350a, dispenser container 400, can be sterilized separately from connected suture 1, by the same or preferably by another sterilization technique or under other energy level, e.g. by Ethylene Oxide based, Gamma irradiation based, X-ray, e-beam irradiation based, thermal based, etc.

Advantageously, sterilizing components containing the medicant is performed by a suitable sterilization technique that does not negatively affect the medicant; while sterilizing suture 10 and needle 100 is performed separately by another suitable sterilization technique.

### SUTURE

In the preferred embodiments, suture 10 comprises any commercially available suture that is approved for use by relevant regulatory authorities. Thus suture 10 maintains desirable mechanical, handling, knot strength, and absorbability properties, corresponding to approved and marketed sutures, after application of medicants according to the present disclosure. Advantageously, suture mechanical and absorption properties are substantially unchanged after application of medicants according to the present disclosure.

Suture 10 can be any suture known in the art, including monofilament or multifilament or braided suture, absorbable or non-absorbable, or partially absorbable suture, made of any biocompatible material, including synthetic or natural materials, and combinations thereof. In some embodiments, polymeric materials of suture 10 comprise bio-resorbable polyester or non-bio-resorbable polypropylene. Particularly preferred as suture 10 are sutures approved for use in surgical procedures, for example sutures sold by Ethicon, Inc. under various brand names, such as VICRYL^{®} (polyglactin 910) Suture, Coated VICRYL^{®} (polyglactin 910) Suture; Coated VICRYL^{®} Plus Antibacterial (polyglactin 910) Suture; VICRYL RAPIDE^{™} (polyglactin 910) Suture; MONOCRYL^{®} (poliglecaprone 25) Suture; PDS^{®} (polydioxanone) Suture; PDS^{®} Plus Antibacterial (polydioxanone) Suture; ETHILON^{®} Nylon Suture; ETHIBOND^{®} Polyester Suture; MERSILENE^{®} Polyester Fiber Suture; PRONOVA^{®} Poly (Hexafluoropropylene - VDF) Suture; PROLENE^{®} Polypropylene Suture; NUROLON^{®} Nylon Suture, etc. Suture 10 can also optionally be a barbed or knotless suture, such as STRATAFIX^{™} Spiral Knotless Tissue Control Device; STRATAFIX^{™} Symmetric Knotless Tissue Control Device, or similar. Suture 10 can be an antimicrobial suture.

Needle 100 can be any surgical suture needle known in the art, including straight or curved needles, needles with various geometries of the needle body and tip or sharp end, needles coated or uncoated with lubricious coatings, and needles made of metal, such as steel, tungsten alloys, etc., or needles made of polymeric materials. Example include suture needles sold by Ethicon, Inc. under various brand names, such as EVERPOINT^{®} Cardiovascular Needle; ETHALLOY^{®} needle alloy based needles; HEMO-SEAL^{™} Needle Suture; ETHIGUARD^{®} Blunt Point Needle; MULTIPLASS^{®} Needles; VISI-BLACK^{™} Surgical Needle. Needles such as taper point, taper cut, cutting edge needles, etc., and similar, can be utilized.

Suture 10 itself can have antimicrobial or any other medically beneficial properties (such as e.g. agents or medicants to assist or stimulate wound healing) or swellable coatings and/or antimicrobial or swellable components incorporated into suture 10 or alternatively can have no antimicrobial or swellable coatings and no antimicrobial or swellable components incorporated into suture 10. Advantageously, the inventive system provides has additional medical activity or antimicrobial activity or swellability properties compared to commercially available and approved sutures, due to the presence of beneficial medicant.

In one embodiment, beneficial medicant applied as shown supra has a minor impact or even no effect on mechanical properties of the resulting suture during and post-installation. Mechanical properties, include, but not limited to, tensile strength, BSR, time to full resorption, and/or elongation under load, and/or breaking strength, and/or knot strength and/or knot slide

Suture 10 materials can be any suture materials known in the art, particularly commercially available and approved suture materials, such as synthetic and natural biocompatible polymeric materials, which may be non-absorbable or absorbable. Examples of synthetic non-absorbable polymeric materials useful to manufacture non-absorbable sutures include polyesters, polyolefins, polyvinylidene fluorides and polyamides. Further examples of non-absorbable materials are polyethylene, polypropylene, nylon, and similar polymers. Examples of synthetic absorbable polymeric materials useful to manufacture absorbable sutures include polymers and copolymers made from lactones such as the lactides, glycolide, p-dioxanone, epsilon-caprolactone, and trimethylene carbonate. Suitable biocompatible, biodegradable polymers may be synthetic or natural polymers. Suitable synthetic biocompatible, biodegradable polymers include polymers selected from the group consisting of aliphatic polyesters, poly(amino acids), copoly(ether-esters), polyalkylene oxalates, tyrosine-derived polycarbonates, poly(iminocarbonates), polyorthoesters, polyoxaesters, polyamidoesters, polyoxaesters containing amine groups, poly(anhydrides), polyphosphazenes, and combinations thereof. For the purposes of this invention aliphatic polyesters include, but are not limited to, homopolymers and copolymers of lactide (which includes lactic acid, D-, L- and meso lactide), glycolide (including glycolic acid), .epsilon.-caprolactone, p-dioxanone (1,4-dioxan-2-one), trimethylene carbonate (1,3-dioxan-2-one), alkyl derivatives of trimethylene carbonate, and blends thereof. Polymers can be selected from poly(lactic acid) (PLA), polyglycolic acid (PGA), polycaprolactone (PCL), polylactide-glycolic acid (PLGA), polypropylene (PP), polyethylene (PE), polydioxanone (PDS), or combinations or copolymers of the monomers thereof. Suitable bioabsorbable, biocompatible elastomeric copolymers include but are not limited to copolymers of .epsilon.-caprolactone and glycolide (preferably having a mole ratio of .epsilon.-caprolactone to glycolide of from about 30:70 to about 70:30, preferably 35:65 to about 65:35, and more preferably 45:55 to 35:65); elastomeric copolymers of .epsilon.-caprolactone and lactide, including L-lactide, D-lactide blends thereof or lactic acid copolymers (preferably having a mole ratio of .epsilon.-caprolactone to lactide of from about 35:65 to about 65:35 and more preferably 45:55 to 30:70) elastomeric copolymers of p-dioxanone (1,4-dioxan-2-one) and lactide including L-lactide, D-lactide and lactic acid (preferably having a mole ratio of p-dioxanone to lactide of from about 40:60 to about 60:40); elastomeric copolymers of .epsilon.-caprolactone and p-dioxanone (preferably having a mole ratio of epsilon-caprolactone to p-dioxanone of from about 30:70 to about 70:30); elastomeric copolymers of p-dioxanone and trimethylene carbonate (preferably having a mole ratio of p-dioxanone to trimethylene carbonate of from about 30:70 to about 70:30); copolymers of trimethylene carbonate and glycolide (preferably having a mole ratio of trimethylene carbonate to glycolide of from about 30:70 to about 70:30); elastomeric copolymer of trimethylene carbonate and lactide including L-lactide, D-lactide, blends thereof or lactic acid copolymers (preferably having a mole ratio of trimethylene carbonate to lactide of from about 30:70 to about 70:30) and blends thereof. In one embodiment, the elastomeric copolymer is a copolymer of glycolide and .epsilon.-caprolactone. In another embodiment, the elastomeric copolymer is a copolymer of lactide and .epsilon.-caprolactone. Non-absorbable, i.e. biodurable suture materials can include nylon, polyethylene, polypropylene or copolymers of the monomers thereof. Suitable biodurable polymers include, but are not limited to polyurethane, polypropylene (PP), polyethylene (PE), polycarbonate, polyamides, such as nylon, polyvinylchloride (PVC), polymethyl-methacrylate (PMMA), polystyrene (PS), polyester, polyetheretherketone (PEEK), polytetrafluoroethylene (PTFE), polytrifluorochloroethylene (PTFCE), polyvinylfluoride (PVF), fluorinated ethylene propylene (FEP), polyacetal, polysulfone, silicones, and combinations thereof.

According to some embodiments, a specialized suture structure is used in combination with the inventive systems to facilitate the coating of the suture and uptake of the medicant. In some embodiments, the suture is core-less braided suture and the internal cavity is configured to uptake the medicant. In some embodiments, suture contains hydrogel, for instance as a coating, wherein hydrogel is swellable and or absorbs medicant. In some embodiments, suture has a lyophilized coating for high absorbency.

In some embodiments, suture can have a hydrophilic and/or surfactant coating, that improves and accelerates wetting and wicking of the suture and pickup of medicant.

In some embodiments, a monofilament suture is provided with microcavities or micro-compartments/microholes or surface roughened/modified to accept and or retain the medicant. The microholes can be drilled under various angles. In some embodiments, the suture has micro-cavities or increased interstices for uptake of the medicant

### Medicants

The antimicrobial agent can be any medicant or drug having antibiotic or antimicrobial function, including chlorhexidine, polyhexamethylene biguanide (PHMB), octenidine, silver particles, silver salts, triclosan, and combinations thereof. Suitable antimicrobial agents may be selected from, but are not limited to, halogenated hydroxyl ethers, acyloxydiphenyl ethers, or combinations thereof. In particular, the antimicrobial agent may be a halogenated 2-hydroxy diphenyl ether and/or a halogenated 2-acyloxy diphenyl ether, esters of acetic acid, chloroacetic acid, methyl or dimethyl carbamic acid, benzoic acid, chlorobenzoic acid, methylsulfonic acid and chloromethylsulfonic acid are particularly suitable. Some particularly advantageous antimicrobial agents are 2,4,4'-trichloro-2'-hydroxydiphenyl ether, commonly referred to as triclosan, chlorhexidine gluconate, and combinations thereof.

In addition to the antimicrobial agents described above, beneficial medicant can be a biocide, a disinfectant and/or an antiseptic, including but not limited to alcohols such as ethanol and isopropanol; aldehydes such as glutaraldehyde and formaldehyde; anilides such as triclorocarbanilide; biguanides such as chlorhexidine; chlorine-releasing agents such as sodium hypochlorite, chlorine dioxide and acidified sodium chlorite; iodine-releasing agents such as povidone-iodine and poloxamer-iodine; metals such as silver nitrate, silver sulfadiazine, other silver agents, copper-8-quinolate and bismuth thiols; peroxygen compounds such as hydrogen peroxide and peracetic acid; phenols; quaternary ammonium compounds such as benzalkonium chloride, cetrimide and ionenes-polyquaternary ammonium compounds.

Beneficial medicant may incorporate drugs such as antibiotics, including but not limited to penicillins such as amoxicillin, oxacillin and piperacillin; cephalosporins parenteral such as cefazolin, cefadroxil, cefoxitin, cefprozil, cefotaxime and cefdinir; monobactams such as aztreonam; beta-lactamase inhibitors such as clavulanic acid sulbactam; glycopeptide such as vancomycin; polymixin; quinolones such as nalidixic acid, ciprofloxacin and levaquin; metranidazole; novobiocin; actinomycin; rifampin; aminoglycosides such as neomycin and gentamicin; tetracyclines such as doxycycline; chloramphenicol; macrolide such as erythromycin; clindamycin; sulfonamide such as sulfadiazine; trimethoprim; topical antibiotics; bacitracin; gramicidin; mupirocin; and/or fusidic acid.

In some embodiments, the medicant further comprises antibodies, growth factors, cytokines , chemokines, wound healing enhancing agents, therapeutic peptides, or any drugs including anticancer, analgesic, etc., and combinations thereof. Beneficial medicant can comprise two or more different medicants incorporated therein.

While the invention has been described above with reference to specific embodiments thereof, it is apparent that many changes, modifications, and variations can be made without departing from the inventive concept disclosed herein.

## Claims

1. A suturing system, comprising:
An elongated flexible suture (10) having a connecting end attached to a needle (100) and an opposing free end (10a), said suture co-packaged with a pierceable container (200) containing a medicant solution,
**characterized in that** said pierceable container further contains a sponge (55) wetted with said medicant solution.

2. The suturing system of claim 1, wherein said suture is absorbable, non-absorbable, monofilament, braid, barbed, or combinations thereof.

3. The suturing system of claim 1, wherein said medicant is anti-microbial or anti-bacterial agent.

4. The suturing system of claim 3, wherein said medicant comprises chlorhexidine, polyhexamethylene biguanide, octenidine, silver particles, silver salts, triclosan, and combinations thereof.

5. The suturing system of claim 1, wherein said pierceable container comprises a pierceable septum (212).

6. The suturing system of claim 3, wherein said medicant solution comprises a rapidly evaporating solvent, optionally wherein said solvent is ethanol.

7. The suturing system of claim 1, wherein said suture is a coreless braid having a hollow core.

8. The suturing system of claim 1, wherein said suture is a monofilament suture, having surface features configured to increase uptake of said medicant solution by said suture, optionally wherein said surface features comprise grooves, apertures, cavities, surface roughening features, and combinations thereof.

9. The suturing system of claim 1, wherein said suture comprises a coating configured to increase uptake of said medicant solution by said suture.

10. The suturing system of claim 1, wherein said suture comprises a coating reactive with any of ingredients of said medicant solution increasing uptake of said medicant by said suture.

11. The suturing system of claim 1, wherein said container contains a first medicant a first combination of medicants in said medicant solution, and said suture is pre-coated by a second medicant, said second medicant can be the same or different from said first medicant or said first combination of medicants.

12. A method of coating the suture of the suturing system of claim 1, comprising the steps of:
1) Providing a suturing system according to claim 1;
2) Gripping the needle manually or with a suture gripper;
3) Piercing said pierceable container with said needle;
4) Entering said pierceable container with said needle and pulling said needle through said pierceable container;
5) Exiting said pierceable container with said needle in a different spot;
6) Pulling said suture through said pierceable container behind said needle, thus exposing said suture to said medicant solution inside said pierceable container;
7) Removing all suture from said pierceable container thus forming suture wetted with said medicant solution and retaining at least some medicant on said suture; and Optionally allowing at least a portion of said solution to evaporate.

13. The method of claim 12, wherein pulling said suture through said pierceable container behind said needle, results in removing an excess of said medicant solution from said suture as said suture exits said pierceable container through interaction of said suture with a wall of said pierceable container or with a pierceable septum closing said pierceable container.

14. The methods of any one of claims 12 to 13 comprising sterilizing said suture and said container separately, optionally wherein said suture and said pierceable container are sterilized by a different sterilization technique.

## Patentansprüche

1. Nähsystem, umfassend:
einen langen flexiblen Faden (10) mit einem Verbindungsende, das an einer Nadel (100) befestigt ist, und einem gegenüberliegenden freien Ende (10a), wobei der Faden zusammen mit einem durchstechbaren Behälter (200) verpackt ist, der eine Pharmakonlösung enthält,
**dadurch gekennzeichnet, dass** der durchstechbare Behälter ferner einen Schwamm (55) enthält, der mit der Pharmakonlösung benetzt ist.

2. Nähsystem nach Anspruch 1, wobei der Faden resorbierbar, nichtresorbierbar, monofil, geflochten, mit Widerhaken versehen oder Kombinationen davon ist.

3. Nähsystem nach Anspruch 1, wobei das Pharmakon ein antimikrobielles oder antibakterielles Mittel ist.

4. Nähsystem nach Anspruch 3, wobei das Pharmakon Chlorhexidin, Polyhexamethylenbiguanid, Octenidin, Silberpartikel, Silbersalze, Triclosan und Kombinationen davon umfasst.

5. Nähsystem nach Anspruch 1, wobei der durchstechbare Behälter ein durchstechbares Septum (212) umfasst.

6. Nähsystem nach Anspruch 3, wobei die Pharmakonlösung ein schnell verdunstendes Lösungsmittel umfasst, optional wobei das Lösungsmittel Ethanol ist.

7. Nähsystem nach Anspruch 1, wobei der Faden ein kernloses Geflecht mit einem hohlen Kern ist.

8. Nähsystem nach Anspruch 1, wobei der Faden ein monofiler Faden ist, der Oberflächenmerkmale aufweist, die konfiguriert sind, um die Aufnahme der Pharmakonlösung durch den Faden zu erhöhen, optional wobei die Oberflächenmerkmale Rillen, Öffnungen, Hohlräume, Oberflächenaufrauhungsmerkmale und Kombinationen davon umfassen.

9. Nähsystem nach Anspruch 1, wobei der Faden eine Beschichtung umfasst, die konfiguriert ist, um die Aufnahme der Pharmakonlösung durch den Faden zu erhöhen.

10. Nähsystem nach Anspruch 1, wobei der Faden eine Beschichtung umfasst, die mit beliebigen Bestandteilen der Pharmakonlösung reaktiv ist, wodurch die Aufnahme des Pharmakons durch den Faden erhöht wird.

11. Nähsystem nach Anspruch 1, wobei der Behälter ein erstes Pharmakon eine erste Kombination von Pharmakon in der Pharmakonlösung enthält und die Naht mit einem zweiten Pharmakon vorbeschichtet ist, wobei das zweite Pharmakon gleich oder verschieden von dem ersten Pharmakon oder der ersten Kombination von Pharmakonen sein kann.

12. Verfahren zum Beschichten des Fadens des Nähsystems nach Anspruch 1, umfassend die Schritte:
1) Bereitstellen eines Nähsystems nach Anspruch 1;
2) Greifen der Nadel manuell oder mit einem Fadengreifer;
3) Durchstechen des durchstechbaren Behälters mit der Nadel;
4) Einführen der Nadel in den durchstechbaren Behälter und Durchziehen der Nadel durch den durchstechbaren Behälter;
5) Verlassen des durchstechbaren Behälters mit der Nadel an einer anderen Stelle;
6) Ziehen des Fadens durch den durchstechbaren Behälter hinter der Nadel, wodurch der Faden der Pharmakonlösung im Inneren des durchstechbaren Behälters ausgesetzt wird;
7) Entfernen des gesamten Fadens aus dem durchstechbaren Behälter, wodurch ein mit der Pharmakonlösung benetzter Faden gebildet wird und mindestens etwas Pharmakon an dem Faden zurückbehalten wird; und optionales Ermöglichen, dass mindestens ein Teil der Lösung verdunstet.

13. Verfahren nach Anspruch 12, wobei das Ziehen des Fadens durch den durchstechbaren Behälter hinter der Nadel dazu führt, dass ein Überschuss der Pharmakonlösung von dem Faden, wenn der Faden den durchstechbaren Behälter verlässt, durch Wechselwirkung des Fadens mit einer Wand des durchstechbaren Behälters oder mit einem durchstechbaren Septum, das den durchstechbaren Behälter verschließt, entfernt wird.

14. Verfahren nach einem der Ansprüche 12 bis 13, umfassend das separate Sterilisieren des Fadens und des Behälters, wobei optional der Faden und der durchstechbare Behälter durch eine unterschiedliche Sterilisationstechnik sterilisiert werden.

## Revendications

1. Système de suture, comprenant :
Une suture souple allongée (10) ayant une extrémité de liaison fixée à une aiguille (100) et une extrémité libre opposée (10a), ladite suture étant co-conditionnée avec un récipient perçable (200) contenant une solution médicamenteuse,
**caractérisé en ce que** ledit récipient perçable contient en outre une éponge (55) mouillée avec ladite solution médicamenteuse.

2. Système de suture selon la revendication 1, dans lequel ladite suture est résorbable, non résorbable, monofilament, tressée, barbelée, ou des combinaisons de ceux-ci.

3. Système de suture selon la revendication 1, dans lequel ledit médicament est un agent antimicrobien ou antibactérien.

4. Système de suture selon la revendication 3, dans lequel ledit médicament comprend de la chlorhexidine, du polyhexaméthylène biguanide, de l'octénidine, des particules d'argent, des sels d'argent, du triclosan, et des combinaisons de ceux-ci.

5. Système de suture selon la revendication 1, dans lequel ledit récipient perçable comprend un septum perçable (212).

6. Système de suture selon la revendication 3, dans lequel ladite solution médicamenteuse comprend un solvant à évaporation rapide, facultativement dans lequel ledit solvant est l'éthanol.

7. Système de suture selon la revendication 1, dans lequel ladite suture est une tresse sans âme ayant une âme creuse.

8. Système de suture selon la revendication 1, dans lequel ladite suture est une suture monofilament, ayant des caractéristiques de surface conçues pour augmenter l'absorption de ladite solution médicamenteuse par ladite suture, facultativement dans lequel lesdites caractéristiques de surface comprennent des rainures, des ouvertures, des cavités, des caractéristiques de rugosité de surface, et des combinaisons de celles-ci.

9. Système de suture selon la revendication 1, dans lequel ladite suture comprend un revêtement conçu pour augmenter l'absorption de ladite solution médicamenteuse par ladite suture.

10. Système de suture selon la revendication 1, dans lequel ladite suture comprend un revêtement réactif avec l'un quelconque des ingrédients de ladite solution médicamenteuse augmentant l'absorption dudit médicament par ladite suture.

11. Système de suture selon la revendication 1, dans lequel ledit récipient contient un premier médicament une première combinaison de médicaments dans ladite solution médicamenteuse, et ladite suture est pré-revêtue par un second médicament, ledit second médicament peut être identique ou différent dudit premier médicament ou de ladite première combinaison de médicaments.

12. Procédé de revêtement de la suture du système de suture selon la revendication 1, comprenant les étapes consistant à :
1) Fournir un système de suture selon la revendication 1 ;
2) Saisir l'aiguille manuellement ou avec un préhenseur de suture ;
3) Percer ledit récipient perçable avec ladite aiguille ;
4) Pénétrer ledit récipient perçable avec ladite aiguille et tirer ladite aiguille à travers ledit récipient perçable ;
5) Sortir dudit récipient perçable avec ladite aiguille à un endroit différent ;
6) Tirer ladite suture à travers ledit récipient perçable derrière ladite aiguille, exposant ainsi ladite suture à ladite solution médicamenteuse à l'intérieur dudit récipient perçable ;
7) Retirer toute suture dudit récipient perçable, permettant ainsi de former une suture mouillée avec ladite solution médicamenteuse et de retenir au moins une partie du médicament sur ladite suture ; et Facultativement, laisser s'évaporer au moins une partie de ladite solution.

13. Procédé selon la revendication 12, dans lequel le fait de tirer ladite suture à travers ledit récipient perçable derrière ladite aiguille entraîne l'élimination d'un excès de ladite solution médicamenteuse de ladite suture lorsque ladite suture sort dudit récipient perçable par interaction de ladite suture avec une paroi dudit récipient perçable ou avec un septum perçable fermant ledit récipient perçable.

14. Procédés selon l'une quelconque des revendications 12 à 13, comprenant la stérilisation de ladite suture et dudit récipient séparément, facultativement dans lesquels ladite suture et ledit récipient perçable sont stérilisés par une technique de stérilisation différente.
